# EUROPEAN PATENT SPECIFICATION

(11) **EP 3 642 331 B1**
(45) Date of publication and mention of the grant of the patent: **24.05.2023**
(21) Application number: 18735229.9
(22) Date of filing: 21.06.2018
(51) Int. Cl.: C12N 5/0783, C12N 5/0784, A61K 35/17

(54) **T-CELL EXPANSION METHOD AND USES**
T-ZELLEN-EXPANSIONSVERFAHREN UND VERWENDUNGEN
PROCÉDÉ ET APPAREIL D'EXPANSION DE LYMPHOCYTES T

(30) Priority: 22.06.2017 GB 201710023; 16.10.2017 GB 201716978
(43) Date of publication of application: 29.04.2020
(73) Proprietor: NEOGAP Therapeutics AB, 171 76 Stockholm (SE)
(72) Inventor: GRÖNLUND, Hans, 18138 Lidingö (SE)
(74) Representative: Abel & Imray LLP
(86) International application number: PCT/EP2018/066690
(87) International publication number: WO 2018/234516

(56) References cited:
- EP-A1- 3 182 125
- WO-A1-2009/076099
- WO-A1-2011/053331
- WO-A1-2016/040900
- WO-A1-2016/053339
- WO-A1-2017/087692
- WO-A2-2008/019366
- WO-A2-2016/077215
- WO-A2-2016/187508
- US-A1- 2016 101 170
- NAM-HYUK CHO ET AL: "A multifunctional core-shell nanoparticle for dendritic cell-based cancer immunotherapy", NATURE NANOTECHNOLOGY, vol. 6, no. 10, 11 October 2011 (2011-10-11), pages 675-682, XP055508194, GB ISSN: 1748-3387, DOI: 10.1038/nnano.2011.149
- HAIYAN LI ET AL: "Alpha-alumina nanoparticles induce efficient autophagy-dependent cross-presentation and potent antitumour response", NATURE NANOTECHNOLOGY, vol. 6, no. 10, 18 September 2011 (2011-09-18), pages 645-650, XP055016222, ISSN: 1748-3387, DOI: 10.1038/nnano.2011.153
- HENGFENG YUAN ET AL: "Multivalent bi-specific nanobioconjugate engager for targeted cancer immunotherapy", NATURE NANOTECHNOLOGY, vol. 12, no. 8, 1 May 2017 (2017-05-01), pages 763-769, XP055508114, GB ISSN: 1748-3387, DOI: 10.1038/nnano.2017.69
- MARK YARCHOAN ET AL: "Targeting neoantigens to augment antitumour immunity", NATURE REVIEWS. CANCER, vol. 17, no. 4, 24 February 2017 (2017-02-24), pages 209-222, XP055507793, GB ISSN: 1474-175X, DOI: 10.1038/nrc.2016.154
- T. D. PRICKETT ET AL: "Durable Complete Response from Metastatic Melanoma after Transfer of Autologous T Cells Recognizing 10 Mutated Tumor Antigens", CANCER IMMUNOLOGY RESEARCH, vol. 4, no. 8, 16 June 2016 (2016-06-16), pages 669-678, XP055531679, US ISSN: 2326-6066, DOI: 10.1158/2326-6066.CIR-15-0215

## Description

### TECHNICAL FIELD

The present invention relates to a method for the expansion of anti-tumor T-cells from a subject with cancer, and compositions and uses of anti-tumor T-cells produced according to the method of the invention, in particular in the treatment of cancer.

### BACKGROUND OF THE INVENTION

The adaptive immune system constitutes the branch of the immune system that can specifically adapt and respond to different pathogens or cell-damaging challenges encountered by the organism, as opposed to the innate immune system that responds in a more generic way. The adaptive system includes both humoral immunity, i.e. antibodies secreted by B-cells, and T-cell-mediated immunity. The specificity of the adaptive immune system lies in the B- and T-cell receptors expressed on B- and T-cells. Through an intricate system of mixing of gene segments the body produces an almost infinite variety of B- and T-cells, each expressing a specific receptor for a certain protein or peptide.

T-cells are lymphocytes that form a major part of the adaptive immune system, where they play a central role in cell-mediated immunity. The defining T-cell receptor (TCR) is expressed on the cell surface, and each receptor recognises an antigen derived peptide presented in the context of an MHC (major histocompatibility complex) molecule. Several types of T-cells exist, each having a distinct function in the cellular immune response.

There are two main types of T-cells with different functions. The CD8-positive cytotoxic T-cell will bind peptides presented on the MHC class I receptor (in humans, termed human leukocyte antigen (HLA) class I) on a cell. All nucleated cells express HLA class I. If the presented peptide is considered as foreign (most often a sign of viral infection), the cytotoxic T-cell will kill the cell either by the protein Granzyme B or Perforin. The helper T-cell (Th), expressing the surface marker CD4, does not kill, rather it orchestrates immune responses by secretion of cytokines, proteins that will augment both pro-inflammatory and sometimes inhibitory signals between cells. Another important function of T-helper cells is to induce class switching of B-cells, e.g. to turn an IgM-secreting B-cell into an IgG-secreting cell, which will increase the humoral immune response against an antigen.

A T-helper cell will by way of its TCR bind its corresponding peptide presented on MHC-II (in humans, termed HLA class II), a receptor specifically expressed on so-called antigen presenting cells (APCs) or endothelial cells. The T-cell activation is dependent on the microenvironment where the Th-APC interaction takes place and the type of so-called costimulatory molecules that are expressed on the APC. The T-helper cell can differentiate into different Th-subsets, e.g. pro-inflammatory Th1, Th2, Th17 cells or to an inhibitory T-helper cell type called regulatory T-cells (Treg). The latter subset is of great importance to control immune responses, since an unrestrained immune system is harmful and can lead to tissue damage and autoimmunity.

Immunologic approaches for the treatment of cancer are known. There has been much success in using monoclonal antibodies targeting checkpoints of immune activation, for example in the treatment of melanoma, lung cancer, bladder cancer and gastrointestinal cancers. Although different monoclonal antibodies have different mechanisms of action, they all lead to the activation and expansion of anti-tumor (or "tumor reactive") T-cells.

As T-cells are often the final effector of immune-mediated cancer treatments, strategies that directly use anti-tumor T-cells have been developed. One approach is adaptive cell transfer (ACT), wherein T-cells are expanded outside the body and re-infused in large numbers into a subject having cancer. This approach is discussed, for example in Klebanoff et al., Nature Medicine, 2016, 22, 26-36.

There have been some successes in this field. For example, a study in which patients with advanced colon cancer were treated using an adoptive immunotherapy protocol was reported by Karlsson et al., Ann Surg Oncol., 2010, 17(7):1747-57. The treatment was based on the isolation and *in vitro* expansion of autologous tumor-reactive lymphocytes isolated intraoperatively from the first lymph node that naturally drains the tumor (the sentinel node). Sentinel node acquired lymphocytes were collected, activated, expanded against autologous tumor extract and returned as a transfusion. No toxic side effects or other adverse effects were observed. Total or marked regression of the disease occurred in four patients with liver and lung metastases and twelve patients displayed partial regression or stable disease. It was found that there was a dose dependent response correlation and stage IV patients displayed a significantly increased survival of 2.6 years compared to control patients 0.8 years. It was concluded that it was possible to expand freshly isolated sentinel node acquired lymphocytes and safely transfuse them back into the patient without complications.

However to obtain enough anti-tumor T-cells for use in adaptive cell transfer using current methods is challenging: surgical removal of a cancer is required in order to obtain tumor-infiltrating lymphocyctes to be expanded. This is an invasive procedure. Furthermore, the cells obtained are few and are frequently unresponsive (anergic) due to immunosuppressive mechanisms from the tumor. This can lead to long periods being necessary for sufficient expansion to be obtained (several months).

Genetically engineered T-cells have been developed to overcome the practicalities that limit the use of adaptive cell transfer using tumor-infiltrating lymphocyctes. Genetically engineered T-cells may be obtained by genetically redirecting a T-cell specificity towards a patient's cancer by introduction of antigen receptors or by introducing a synthetic recognition structure termed a "chimeric antigen receptor" into a T-cell. Although genetically engineered T-cells have found success in treating hematologic cancers, the safety and selectivity of genetically engineered T-cells for treating solid cancers requires improvement. WO 2016/053339 (The United States of America, as Represented by the Secretary, Department of Health and Human Services) discloses a method of isolating T-cells having antigenic specificity for a mutated amino acid sequence encoded by a cancer-specific mutation, the method comprising: identifying one or more genes in the nucleic acid of a cancer cell of a patient, each gene containing a cancer-specific mutation that encodes a mutated amino acid sequence; inducing autologous APCs of the patient to present the mutated amino acid sequence; co-culturing autologous T-cells of the patient with the autologous APCs that present the mutated amino acid sequence; and selecting the autologous T-cells. The selection step requires selecting the autologous T-cells that (a) were co-cultured with the autologous APCs that present the mutated amino acid sequence and (b) have antigenic specificity for the mutated amino acid sequence presented in the context of a major histocompatability complex (MHC) molecule expressed by the patient to provide isolated T-cells having antigenic specificity for the mutated amino acid sequence encoded by the cancer-specific mutation. It is disclosed that once those cells are selected, they are expanded by culturing with feeder PBMCs (e.g. irradiated allogeneic PBMC), interleukin (IL)-2, and OKT3 antibody.

To induce autologous APCs of the patient to present the mutated amino acid sequence, WO2016/053339 teaches to use pulsing with free peptide or nucleotide constructs to introduce the neoantigen (or neoantigen coding material) into a cell. The pulsing with free peptide introduces the neoantigen into the cytosol (intracellular fluid) of the cell.

EP3182125 relates to the field of diagnostic and analytic methods involving determination of antigen-specific T-cell activation.

Thus, there remains need for improved methods for providing anti-tumor T-cells suitable for use in the treatment of cancers, with good safety and selectivity, and that can be practically used in a clinical setting.

### SUMMARY OF THE INVENTION

The present invention provides a method for the expansion of anti-tumor T-cells from a subject with cancer, and a composition comprising anti-tumor T-cells from a subject with cancer, as set out in the appended claims.

The inventors surprisingly found that phagocytosable particles having tumor neoantigenic constructs tightly associated thereto can be internalised by antigen-presenting cells, that the tumor neoantigenic constructs can then be presented on the surface of those cells, and bring about expansion of T-cells being cultured in the presence of the particles/APC mixture. Use of the phagocytosable particles leads to surprisingly high uptake and processing of the tumor neoantigenic constructs.

By using a phagocytosable particle, having one or more tumor neoantigenic constructs tightly associated thereto, in combination with antigen-presenting cells, and exposing the particle/antigen-presenting cells to a T-cell sample from a subject with cancer, a high level of expansion of anti-tumor T-cells can be achieved. In general, a plurality of particles is used, for example 100 to 1×10⁷ particles (which may all be the same species, or which may be different, as described in further detail below).

The method is especially effective as it allows the sequence of the tumor neoantigenic construct to be controlled, and also allows for more than one tumor neoantigenic construct to be included on a particle. For example different tumor neoantigenic constructs comprising one or more different neoantigen epitopes can be attached to a particle. This multiplex capability of the particles allows stimulation with multiple epitopes to maximise T-cell expansion.

The use of a phagocytosable particle also allows a large amount of tumor neoantigenic construct to enter the cell and be presented on the cell surface. A single phagocytosable particle typically has 0.5 to 1 million tumor neoantigenic constructs associated with it. This leads to high levels of surface expression of neoantigen on APC's that have internalised a phagocytosable particle.

The use of a phagocytosable particle also surprisingly enables the tumor neoantigenic constructs to be presented to the antigen-presenting cells in a highly sterile and pure form. For example, phagocytosable particles are particularly effective in enabling the removal of contaminants, such as pyrogens.

Without wishing to be bound by any particular theory, it is believed that phagocytosable particles having tumor neoantigenic constructs tightly associated are especially effective because they are internalised by antigen-presenting cells by phagocytosis into a phagosome. The tumor neoantigenic constructs are then cleaved from the particles in the phagosome and fragments of the tumor neoantigenic construct are presented on the surface of the antigen-presenting cells via the major histocompatibility (MHC) class II pathway and presented on the cell surface by a MHC class II molecule. It is also believed that this is not the exclusive process for the antigen to be presented on the APCs, and that some fragments of the tumor neoantigenic construct may also be presented on the surface of the antigen-presenting cells via the major histocompatibility (MHC) class I pathway and presented on the cell surface by a MHC class I molecule, in a process known as cross-presentation. Thus, although it is expected that fragments of the tumor neoantigenic construct are presented on APCs predominantly via the MHC class II pathway, it is expected that some will be presented via the MHC class I pathway, and so the method of the present invention harnesses both pathways to a varying extent.

When antigens are presented by an MHC class II molecule, they generally activate helper T-cells (also known as CD4+ T-cells), which do not directly kill other cell types, but instead orchestrate immune responses by secretion of cytokines, inducing class switching of B-cells to assist the B-cells to make antibodies and stimulating expansion of other T-cell types. This means that the population of T-cells activated and expanded according to the method of the present invention, when administered to a patient, has the advantage that the immune response starts slowly (thus leading to few side effects), has a long lasting effect, and can target the cancers in many different ways by harnessing the whole immune system (for example, rather than only activating CD8-positive cytotoxic T-cell which can only attack the tumour cells directly). This is in contrast to what would be expected to occur when an antigen is provided as free peptide or a nucleotide construct that expresses the peptide. Such an antigen would be expected to be taken up into the cytosol of an APC, which results in the neoantigen being presented on the cell surface solely via the MHC class I pathway by an MHC class I molecule. This, in turn, results predominantly in the activation of CD8-positive cytotoxic T-cells.

When the composition of the present invention is used for the treatment of cancer, the patient's own immune system is harnessed and stimulated. That means that no chemo-induced depletion of endogenous T-cells is required before administering the T-cells. The method thus has fewer side effects than certain comparable alternative methods.

Furthermore, after the initial targeting, memory T-cells derived from the T-helper cells remain in circulation and they can mount a rapid and effective secondary immune response for as long as cancer cells expressing the neoantigen remain in the body, or if the same cancer returns.

Thus, a population of T-cells expanded according to the method of the present invention, when expanded and administered to a patient, will continue to work even after the original T-cells administered have died, due to the memory T-cells derived from the originally activated T-cells.

The method of the invention also enables the expanded T-cells to be generated very rapidly, as the phagocytosable particle with one or more tumor neoantigenic constructs tightly associated thereto can be prepared rapidly in sterile and pure form, and the expansion can then be commenced and completed rapidly. This is important as the success of the therapy relies on the cancer in the subject being essentially the same when the T-cells are administered as it was when the sample was taken from the subject.

### BRIEF DESCRIPTION OF THE FIGURES

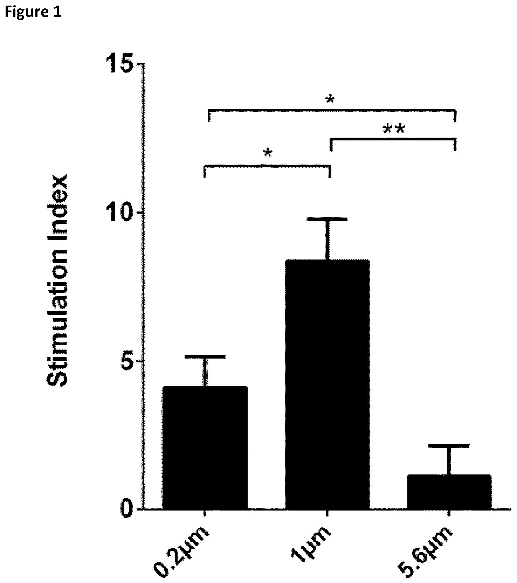

Figure 1. Effect of particle size on T-cell activation. Proliferation assay (with thymidine incorporation) with splenocytes from ovalbumin sensitized mice. Comparison of ovalbumin coupled to differently sized particles with a diameter of 5.6µm, 1µm and 0.2µm. P-values determined using students T-test and written indicated when p<0.05 found. Staples denote SD.

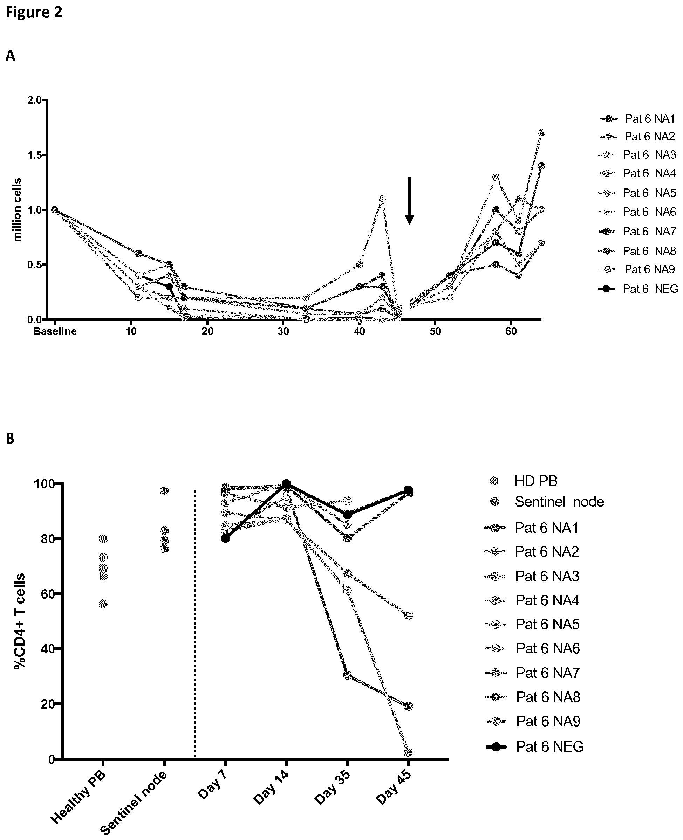

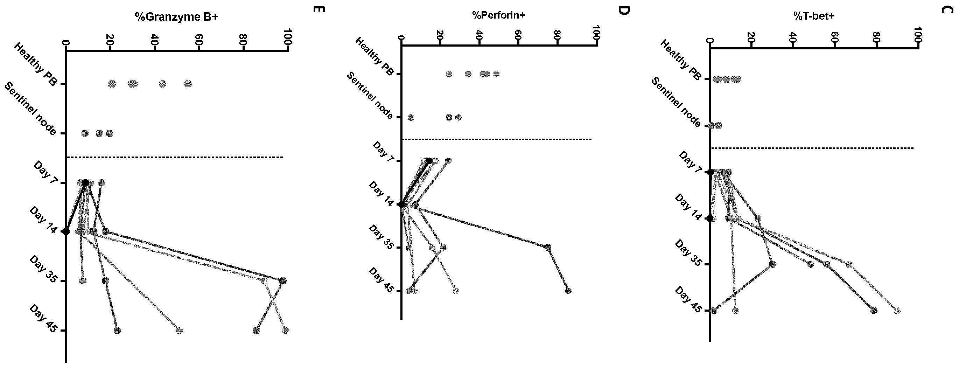

Figure 2. Expansion of T-cells by stimulation with a neo-antigen peptides NA1-9. Figure 2A shows the number of cells in the culture over time. Figure 2B shows the %CD4+/total T-cells.
Figure 2C shows the T-bet expression in CD4. Figure 2D shows the expression of granzyme B and perforin in CD8+ T-cells.

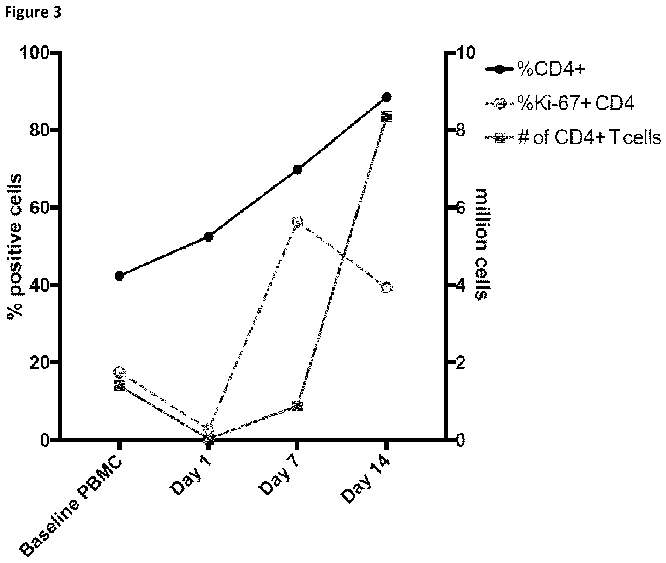

Figure 3. Expansion of T-cells by stimulation with a neo-antigen construct. Percent among T-cells (small squares) and total number (large squares) of CD4+ T-cells, as well as proliferating CD4+ cells (circles) are shown.

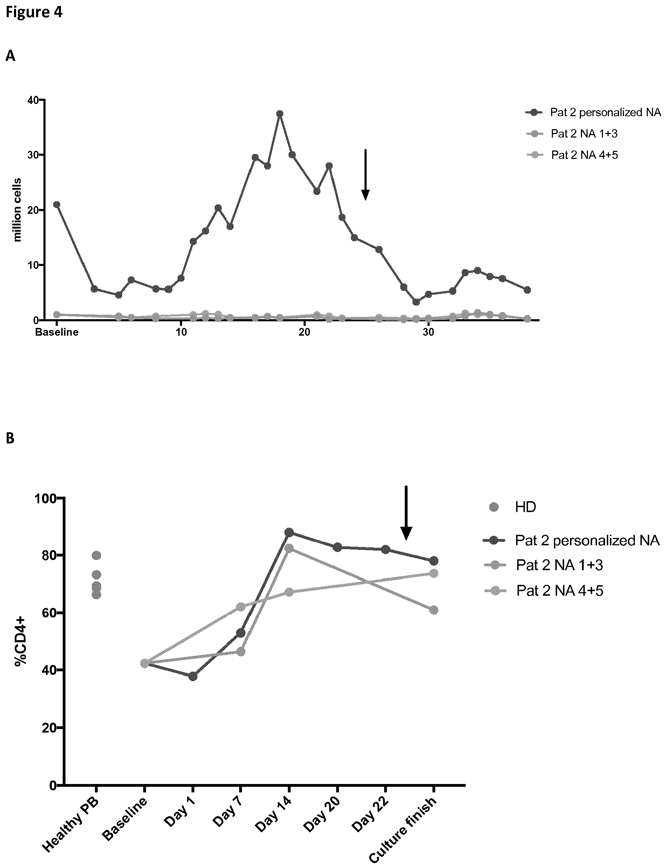

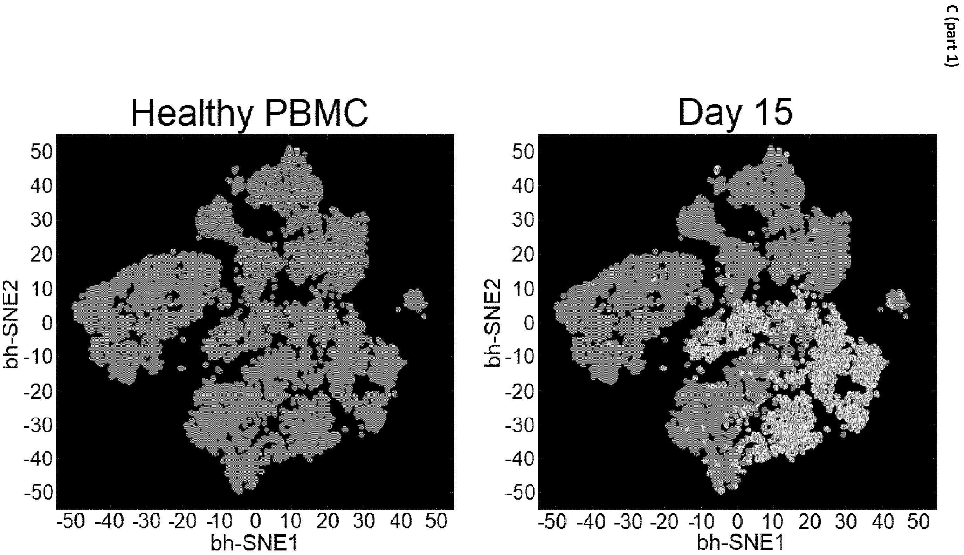

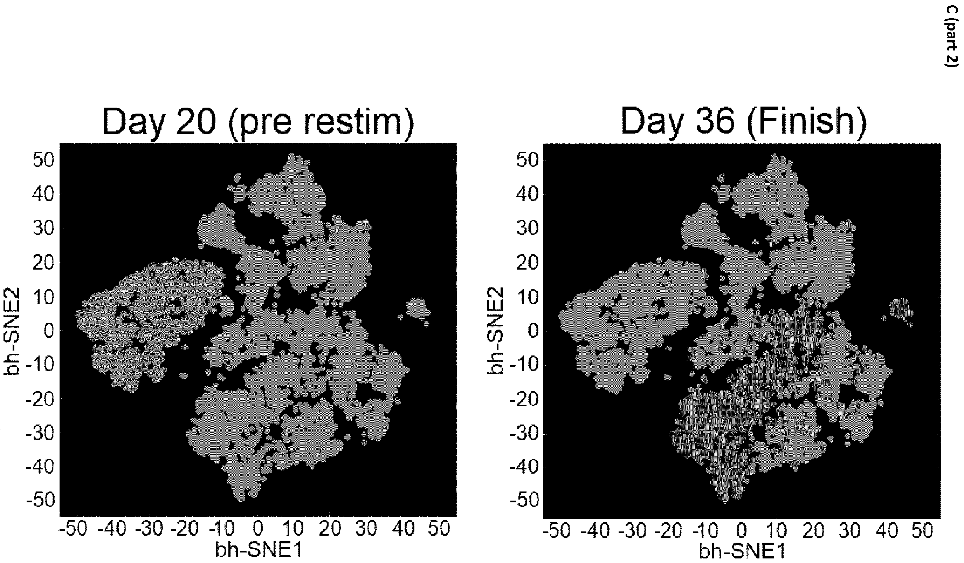

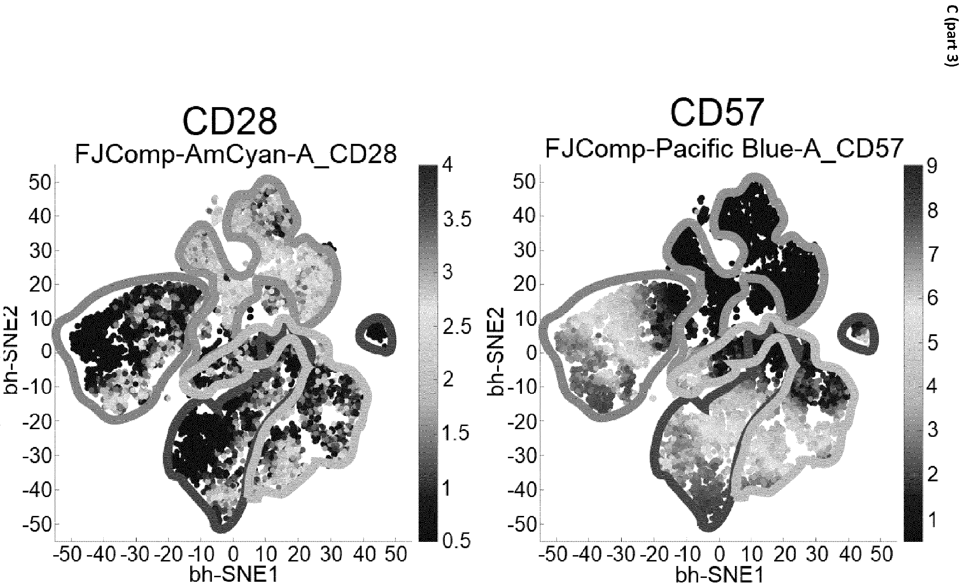

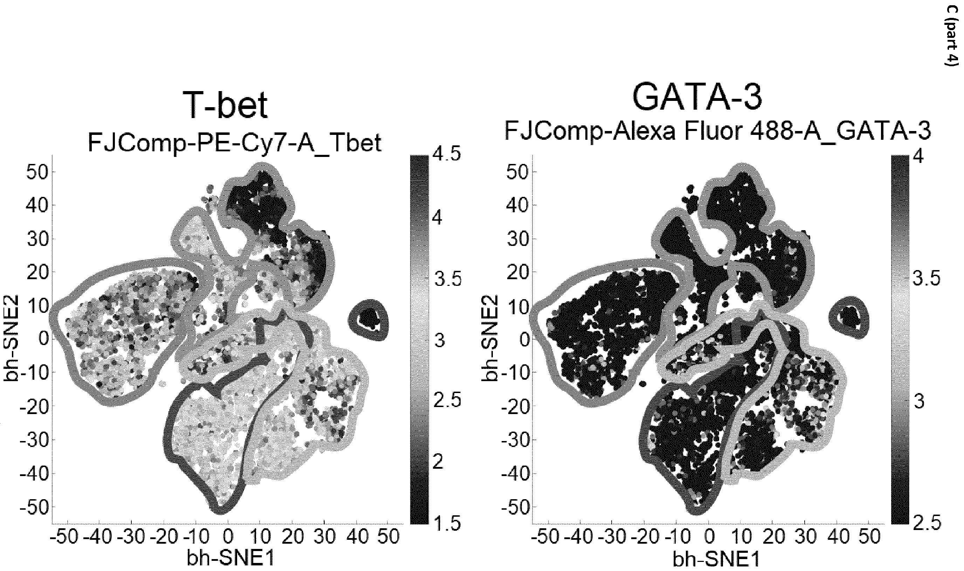

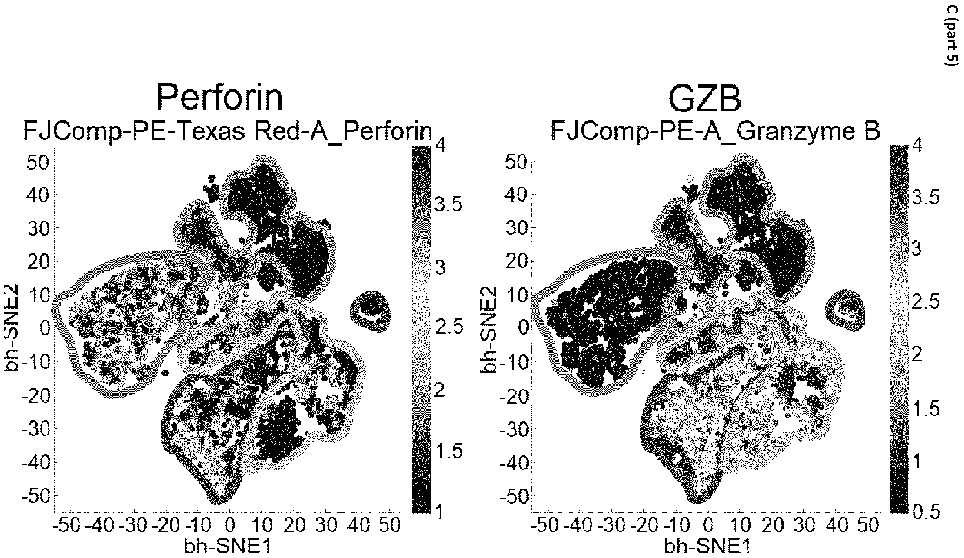

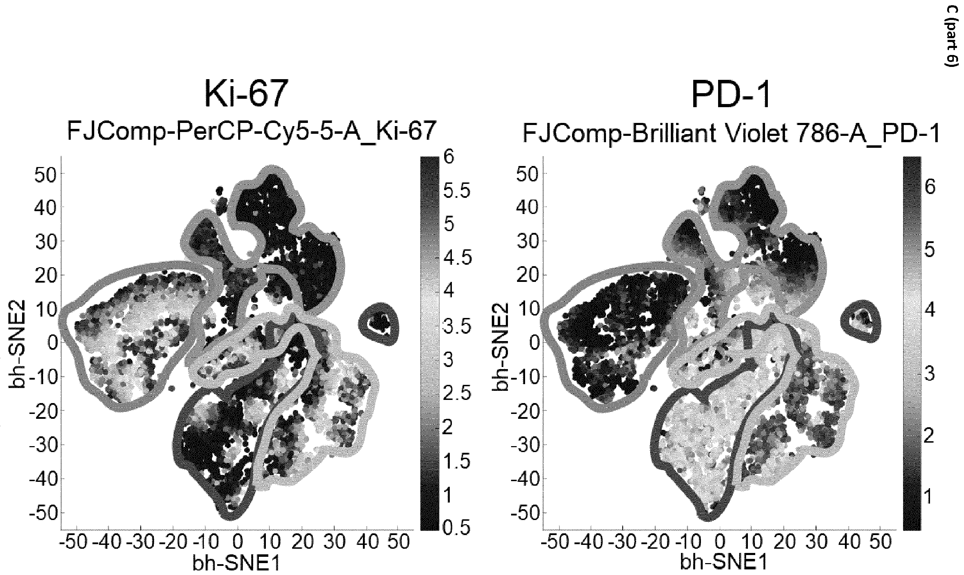

Figure 4. Expansion of T-cells by stimulation with a neo-antigen construct. Figure 4A shows the number of cells in the culture over time (red line: personalized peptide; pink and orange lines: control cultures predicted peptides with the same protein but with the predicted mutations (NA1, 3, 4 and 5)). Figure 4B shows the %CD4+/total T-cells. Figure 4C visualizes an analysis performed with the Barnes-Hut Stochastic Neighbor Embedding (BH-SNE) algorithm for CD4 T-cells, where all cells in the samples are clustered on a 2-dimentional map according to the similarity in expression intensity according to a set of chosen markers: CD28, CD57, T-bet, GATA-3, Perforin, Granzyme B (GZB), Ki-67 and PD-1.

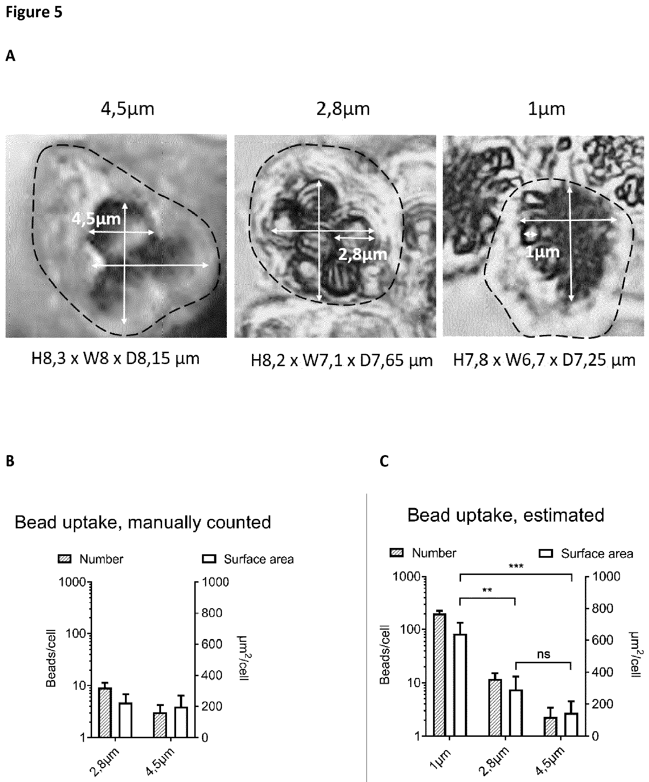

Figure 5A. Confocal microscope images of PBMCs with intracellular phagocytosed antigen coupled particles of three sizes (4.5 µm, 2.8 µm or 1 µm) after incubation for 18 h at 37 °C with the antigen coupled particles.
Figure 5B. Graph showing uptake of antigen coupled particles of two sizes (4.5 µm or 2.8 µm) in PBMCs after incubation for 18 h at 37 °C, as assessed by manual counting.
Figure 5C. Graph showing uptake of antigen coupled particles of three sizes (4.5 µm, 2.8 µm or 1 µm) in PBMCs after incubation for 18 h at 37 °C, as assessed by volume calculation (*p<0,05 **p<0,01 ***p<0,001, calculated using Students T-test).

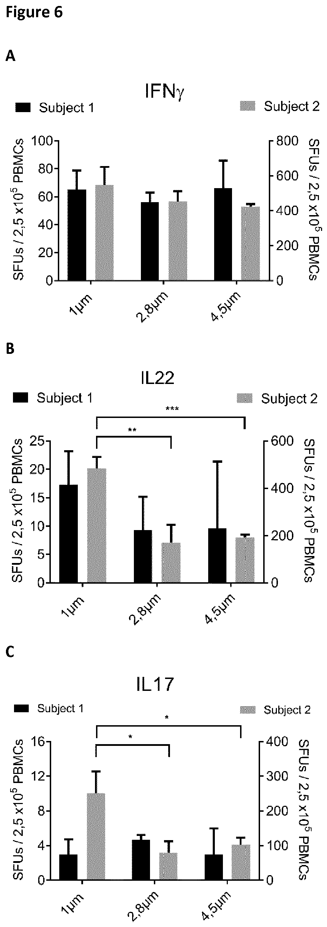

Figure 6A. Graph showing the relative increase in the level of IFNy-production in PBMCs from CMV-sensitive healthy donors (n=2) stimulated with the antigen coupled particles of three sizes (4.5 µm, 2.8 µm or 1 µm) compared to non-stimulated cells, as assessed in the FluoroSpot assay of Example 3a(iv).
Figure 6B. Graph showing the relative increase in the level of IL22-production in PBMCs from a CMV-sensitive healthy donor (n=1) stimulated with the antigen coupled particles of three sizes (4.5 µm, 2.8 µm or 1 µm) compared to non-stimulated cells, as assessed in the FluoroSpot assay of Example 3a(iv).
Figure 6C. Graph showing the relative increase in the level of IL17-production in PBMCs from a CMV-sensitive healthy donor (n=1) stimulated with the antigen coupled particles of three sizes (4.5 µm, 2.8 µm or 1 µm) compared to non-stimulated cells, as assessed in the FluoroSpot assay of Example 3a(iv).

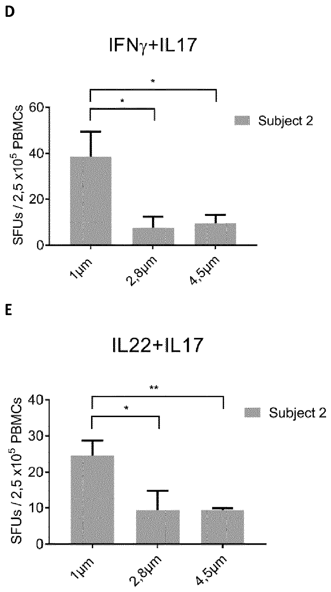

Figure 6D. Graph showing the relative increase in the dual-cytokine production of IFNy and IL17 in PBMCs from a CMV-sensitive healthy donor (n=1) stimulated with the antigen coupled particles of three sizes (4.5 µm, 2.8 µm or 1 µm) compared to non-stimulated cells, as assessed in the FluoroSpot assay of Example 3a(iv).
Figure 6E. Graph showing the relative increase in the dual-cytokine production of IL22 and IL17 in PBMCs from a CMV-sensitive healthy donor (n=1) stimulated with the antigen coupled particles of three sizes (4.5 µm, 2.8 µm or 1 µm) compared to non-stimulated cells, as assessed in the FluoroSpot assay of Example 3a(iv).

### DEFINITIONS

*Endotoxins,* e.g. Lipopolysaccharide (LPS), comprise covalently linked lipid and polysaccharide subunits found on the outer cell wall of gram-negative bacteria, such as Escherichia coli.

*CD4+ T-cells* (or T-helper cells or CD4+ helper T-cells) are cells that orchestrate immune responses through cytokine secretion. They can both supress or potentiate other immune cells, such as stimulate antibody class switching of B-cells, expansion of cytotoxic T-cells or potentiate phagocytes. They get activated by antigen presentation via MHC class II on APCs and they express a T-cell receptor (TCR) specific for a stretch of approximately 15 amino acids (a so-called T-cell epitope) within a particular antigen.

*CD8+ T-cells* (or cytotoxic T-cells) are cells that kill tumor cells, infected cells or cells otherwise damaged. Unlike CD4+ T-cells they do not need APCs for activation. Their T-cell receptor recognizes antigen derived peptides (approximately 7-10, for example 8, amino acids long) presented by MHC class I, a protein expressed on all nucleated cells.

*Antigen-specific T-cell activation* is a process requiring interaction between the TCR and a defined peptide presented on a MHC (HLA) molecule in combination with co-stimulation.

*Antigen-presenting cells (APCs)* are typically dendritic cells (DCs), B-cells or macrophages, cells that either phagocytose or internalise extra-cellular organisms or proteins, i.e. antigens, and after processing present antigen-derived peptides on MHC class II to CD4+ T-cells. In blood, monocytes are the most abundant antigen-presenting cells.

*A phagocytosable particle* is defined as a particle able to be phagocytosed by cells of the immune system, in particular monocytes.

*Peripheral blood mononuclear cells (PBMCs*), is a fraction of human blood prepared by density gradient centrifugation of whole blood. The PBMC fraction mainly consists of lymphocytes (70-90%) and monocytes (10-30%), while red blood cells, granulocytes and plasma have been removed. Monocytes may in some instances make up 10 to 20% of the cell numbers in a PBMC sample, for example 10 to 15%.

*A tumor neoantigen* is a tumor induced change in the sequence of amino-acids of a peptide or protein that may be recognized by the immune system as a foreign material.

An *amino-acid sequence comprising a mutated amino-acid* is an amino-acid sequence of a peptide or protein in a tumor cell of a subject with an alteration compared to the sequence in a non-tumor cell in a subject. The mutated amino-acid may be a point mutation: for example the insertion or deletion of an amino acid, or the substitution of a single amino acid for a different amino acid in a sequence of protein or peptide. In some circumstances, two or more mutated amino acids may be present in a row. A mutation may be a frame-shift mutation.

### DETAILED DESCRIPTION

The present invention provides methods for the expansion of anti-tumor T-cells, and compositions and uses of anti-tumor T-cells produced according to the method of the invention, as disclosed in more detail below.

### The phagocytosable particle and its properties:

The particle is phagocytosable by an antigen-presenting cell (APC). The APCs can phagocytose particles of many different materials and shapes. The size of the particles thus needs to be chosen to allow for phagocytosis. A particle that is too small of a size may not trigger phagocytosis by a particular APC. A particle that is too large of a size may not be phagocytosable by a particular APC as it may not fit in the cell.

Preferably, the particles for use in a method of the invention may have a largest dimension of less than 5.6 µm, preferably less than 4 µm, more preferably less than 3 µm, for example less than 2.5 µm, less than 2 µm or less than 1.5 µm. Preferably, the particles for use in a method of the invention may have a largest dimension of greater than 0.001 µm, preferably greater than 0.005 µm, preferably greater than 0.01 µm, preferably greater than 0.05 µm, preferably greater than 0.1 µm, more preferably greater than 0.2 µm, and even more preferably greater than 0.5 µm. Preferably the particles have a largest dimension in the interval 0.1-5.6 µm, preferably 0.2-5.6 µm, preferably 0.5-5.6 µm, preferably 0.1-4 µm, preferably 0.5-4 µm, more preferably 0.1-3 µm, more preferably 0.5-3 µm, even more preferably 0.1-2.5 µm, even more preferably 0.5-2.5 µm, even more preferably 0.2-2 µm, even more preferably 0.5-2 µm, more preferably 1-2 µm , or for example about 1 µm , about 1.5 µm or about 2 µm (preferably about 1 µm). The particles may be substantially spherical, in which case the dimensions would refer to diameter. Preferably the particles are substantially spherical.

The preferred particle sizes are those large enough to enter a cell by phagocytosis, but small enough that more than one particle can enter the same cell by phagocytosis. Having more than one phagocytosed particle means that the APC can take peptides from several particles in different phagosomes at the same time, and thus will maximise expression of the neoantigen on the cell surface via the MHC class II pathway.

In general, a size similar to that of bacteria facilitates complete phagocytosis by APCs. Complete phagocytosis leads to good antigen degradation by APCs and subsequently good presentation to T-cells via MHC class II. The optimal size has been investigated by the current inventors (see Examples 3a and 3b, and Figures 1, 5A-C, and 6A-E).

The particles have paramagnetic properties, for example superparamagnetic properties. A paramagnetic or superparamagnetic particle can be separated completely from the T-cells by use of a magnet. Importantly, the APCs containing an internalised particle can also be separated from the T-cells by use of a magnet. This allows the T-cells to be rapidly isolated from the incubation mixture. The T-cells can then be administered to patients in the knowledge that all particles have been removed. The invention thus provides an especially safe method of expanding T-cells.

A paramagnetic particle also facilitates the sterilisation and/or denaturing of the particles with the tumor neoantigenic construct associated to it. Washes are discussed in further detail below. During a wash, the particles can be collected and/or held in place by a magnet. It is also possible to perform a wash by other means, such as by holding the particles (whether paramagnetic or not) in a column, or sedimenting the particles by gravity or by centrifugation.

An example of a superparamagnetic particle are Dynabeads^{™} (Invitrogen). They are available in various functionalisable forms, for example Dynabeads M-270 Carboxylic acid, Dynabeads M-270 Amine, and Dynabeads MyOne Carboxylic acid. Dynabeads MyOne Carboxylic Acid are uniform, monosized superparamagnetic particles, which are composed of highly cross-linked polystyrene with evenly distributed magnetic material. The particles are further coated with a hydrophilic layer of glycidyl ether, concealing the iron oxide inside them. Carboxylic acid groups are then introduced on the surface of the particles.

Other examples of superparamagnetic particles include Encapsulated Carboxylated Estapor^{®} SuperParamagnetic Microspheres (Merck Chimie S.A.S.) and Sera-Mag SpeedBeads (hydrophilic) Carboxylate-Modified Magnetic Particles (GE Healthcare UK Limited). Encapsulated Carboxylated Estapor^{®} SuperParamagnetic Microspheres are made of a core-shell structure. The superparamagnetic iron oxide material (~40%) is encapsulated by a polystyrene film and does not interfere with components on the surface. Sera-Mag SpeedBeads (hydrophilic) Carboxylate-Modified Magnetic Particles are magnetic particles of uniform size and feature a second layer of magnetite (in total two layers constituting ~60%).

As a result, Sera-Mag Speed Beads respond very quickly to a magnetic field to separate quickly and completely from suspensions. They have a cauliflower-like surface, which increases overall surface area and binding capacity of the particles.

### Association of the antigen polypeptide and the particle

The tumor neoantigenic construct is associated to the particle in a manner that allows performing a sterilisation and denaturing wash as discussed below without dissociating the tumor neoantigenic construct from the particle.

One way of associating the polypeptide to the particle is shown in Example 1. However, the precise manner of association is not critical for the methods of the invention. Preferably, the polypeptide is covalently linked to the particle (for example through an amide bond between an amine group or a carboxylic acid group of the polypeptide and a carboxylic acid group or an amine group on the surface of the particle). Alternatively, the candidate antigen polypeptide may be linked to the particles via a metal chelate. For example, particles linked with a metal chelating ligand, such as iminodiacetic acid can bind metal ions such as Cu²⁺, Zn²⁺, Ca²⁺, Co²⁺ or Fe³⁺. These metal chelates can in turn bind proteins and peptides containing for example histidine or cysteine with great strength. Thus, particles with metal chelates can non-covalently adsorb peptides/proteins, in a manner which allows stringent washing to reduce the amount of LPS and other contaminating components in the bound peptides/proteins.

The particle may comprise polymer, glass or ceramic material (e.g. the particle may be a polymer particle, a glass particle or a ceramic particle). Preferably the particle comprises a synthetic aromatic polymer, such as polystyrene, or, another polymer, such as polyethylene. The particle may comprise a polysaccharide polymer, for example agarose. Preferably, the particle does not comprise a polysaccharide polymer (and preferably is not a polysaccharide polymer particle or a carbohydrate based particle). Preferably the particle is a polystyrene or polyethylene particle, and more preferably is a polystyrene particle. Preferably the particle is a monodispersed particle (i.e. a particle with a uniform size).

### Tumor neoantigenic construct

A tumor neoantigenic construct is a polypeptide that comprises more than one tumor neoantigen and/or at least one mutated amino-acid sequence known or suspected to be expressed in a cancer cell in a subject. The tumor neoantigenic construct may comprise one or more covalently linked peptides, wherein one or more of the covalently linked peptides comprises an amino-acid sequence comprising at least one mutated amino-acid known or suspected to be associated with a cancer in a subject. In certain embodiments of the method of the invention , one or more of the covalently linked peptides comprises an amino-acid sequence comprising at least one mutated amino-acid sequence known or suspected to be expressed in a cancer cell in a subject. The mutated amino-acid sequence known or suspected to be expressed in a cancer cell in a subject may be an amino-acid sequence known or suspected to be expressed by the tissue or organ in which the tumor is present. The amino-acid sequence comprising at least one mutated amino-acid known or suspected to be associated with a cancer in a subject (or comprising a mutated amino-acid sequence comprising at least one amino-acid sequence known or suspected to be expressed in a cancer cell in a subject) may also be referred to as a 'neoantigen epitope'.

Preferably, the mutated amino-acid is a substituted amino acid (i.e. an amino-acid in a protein or peptide in a non-tumor cell is substituted for a different amino acid in a tumor cell). Alternatively, the mutated amino-acid may be an amino acid that has been deleted from the sequence (i.e. deleted in the protein/peptide in the tumor cell compared to the non-tumor cell); or the mutated amino-acid may be an amino acid that has been inserted into the sequence (i.e. a new amino acid has been added to the protein/peptide in the tumor cell compared to the non-tumor cell).

A neoantigen epitope of the tumor neoantigenic construct may be designed by selecting a mutated protein/peptide (or mutated gene) that is known or suspected to be associated with a cancer in a subject; locating the mutated amino acid(s) in the sequence of the protein or peptide (or in the protein/peptide that would result from expression of the gene); and selecting the part of the protein/peptide comprising the mutated amino acid(s), plus a number of flanking amino acids to the C-terminal end and N-terminal end of the mutated amino acid(s). In certain preferred embodiments of the method of the invention, there is a single mutated amino acid in the sequence of the protein or peptide compared to the protein or peptide sequence in a non-tumor cell. More preferably, there is a single substituted mutated amino acid in the sequence of the protein or peptide compared to the protein or peptide sequence in a non-tumor cell.

Preferably the number of flanking amino acids to the C-terminal end of the mutated amino acid(s) is at least 5, preferably at least 8, for example 8, 9, 10, 11 or 12 amino-acids. Preferably the number of flanking amino acids to the N-terminal end of the mutated amino acid(s) is at least 5, preferably at least 8, for example 8, 9, 10, 11 or 12 amino-acids. The number of flanking amino acids to the C-terminal end and N-terminal end of the mutated amino acid(s) may be the same of may be different, preferably the number is the same.

In certain embodiments of the method of the invention, one or more of the covalently linked peptides comprises an amino-acid sequence comprising at least one mutated amino-acid sequence known or suspected to be expressed in a cancer cell in a subject. The mutated amino-acid sequence known or suspected to be expressed in a cancer cell in a subject may be an amino-acid sequence known or suspected to be expressed by the tissue or organ in which the tumor is present.

In embodiments of the method of the invention wherein the tumor neoantigenic construct comprises two or more covalently linked peptides, preferably each covalently linked peptide comprises an amino-acid sequence comprising at least one mutated amino-acid known or suspected to be associated with a cancer in a subject (i.e. a neoantigen epitope). Alternatively, in embodiments of the method of the invention wherein the tumor neoantigenic construct comprises two or more covalently linked peptides, preferably the tumor neoantigenic construct comprises at least one peptide comprising an amino-acid sequence comprising at least one mutated amino-acid known or suspected to be associated with a cancer in a subject (i.e. a neoantigen epitope), and at least one peptide comprising an amino-acid sequence comprising an amino-acid sequence known or suspected to be expressed in a cancer cell in a subject.

In embodiments of the method of the invention wherein the tumor neoantigenic construct comprises two or more covalently linked peptides, the peptides may be directly linked, or linked via a spacer moiety. The spacer moiety may be a short sequence of amino acids, for example 1 to 15 amino acids, preferably 1 to 10 amino acids, and more preferably 1 to 5 amino acids (for example, 1, 2, 3, 4 or 5 amino acids). Preferably, the spacer moiety comprises the motif VVR. In one embodiment of the method of the invention the spacer moiety has the sequence VVR. In an alternative embodiment of the method of the invention, the spacer moiety does not comprise the motif VVR, for example the spacer moiety does not have the sequence VVR. In another embodiment of the method of the invention, the spacer moiety comprises the motif GGS, for example the spacer moiety has the sequence GGS. In another embodiment of the method of the invention, the peptides are directly linked (i.e. there is no spacer moiety, for example no amino acid spacer moiety).

In preferred embodiments of the method of the invention, the tumor neoantigenic construct comprises two or more covalently linked peptides, and more preferably three or more covalently linked peptides (for example three, four, five, six, seven, eight, nine or ten covalently linked peptides, preferably three, four, five, or six covalently linked peptides, and more preferably three, four or five covalently linked peptides). Preferably, the linked peptides are each linked via a spacer moiety. Each linker moiety may be the same or may be different. It has been found that three covalently linked peptides, each comprising an amino-acid sequence comprising at least one mutated amino-acid known or suspected to be associated with a cancer in a subject, is especially effective.

The structure of the tumor neoantigenic construct comprising two linked peptides may be as follows:
- [epitope sequence 1] - [linker moiety] - [epitope sequence 2].

The structure of the tumor neoantigenic construct comprising three linked peptides may be as follows:
- [epitope sequence 1] - [linker moiety] - [epitope sequence 2] - [linker moiety] - [epitope sequence 3].

The structure of the tumor neoantigenic construct comprising four linked peptides may be as follows:
- [epitope sequence 1] - [linker moiety] - [epitope sequence 2] - [linker moiety] - [epitope sequence 3] - [linker moiety] - [epitope sequence 4].

The structure of the tumor neoantigenic construct comprising five linked peptides may be as follows:
- [epitope sequence 1] - [linker moiety] - [epitope sequence 2] - [linker moiety] - [epitope sequence 3] - [linker moiety] - [epitope sequence 5].

The structure of the tumor neoantigenic construct comprising n linked peptides may be as follows:
- [epitope sequence 1] - [linker moiety] - [epitope sequence 2] - [linker moiety] - [epitope sequence 3] ..... - [linker moiety] - [epitope sequence n].

In preferred embodiments of the method of the invention, each peptide of the tumor neoantigenic construct comprises 5 to 50 amino-acids, more preferably 5 to 30 amino acids, more preferably 8 to 30 amino acids, and most preferably 10 to 25 amino acids, for example 10, 11, 12, 13, 14, 15, 16, 17, 18, 19, 20, 21, 22, 23, 24 or 25 amino acids. In one especially preferred embodiment of the method of the invention the each peptide of the tumor neoantigenic construct comprises 17 to 25 amino acids, for example it comprises the mutated amino-acid plus 8 to 12 amino acids (preferably 10 amino acids) to the C-terminal end and N-terminal end of the mutated amino acid.

In embodiments of the method of the invention wherein the particle is associated with two or more tumor neoantigenic constructs, each tumor neoantigenic construct may have the same polypeptide sequence or may have different polypeptide sequences. For example, a first tumor neoantigenic construct may comprise one or more covalently linked peptides (for example, one or more neoantigen epitopes), and a second tumor neoantigenic construct may comprise one or more different covalently linked peptides (for example, one or more different neoantigen epitopes to the neoantigen epitope(s) of the first tumor neoantigenic construct).

A tumor neoantigenic construct may comprise at least 20 amino-acids, more preferably at least 30 amino acids, more preferably at least 40 amino acids, more preferably at least 50 amino acids, even more preferably at least 60 amino-acids. A tumor neoantigenic construct may comprise less than 150 amino acids, preferably less than 130 amino acids, more preferably less than 100 amino acids, even more preferably less than 85 amino acids, and even more preferably less than 75 amino acids.

The method of the present invention allows for more than one tumor neoantigenic construct to be associated with a particle. A tumor neoantigenic construct may comprise one or more neoantigen epitopes. Use of several neoantigen epitopes on a particle increases the chance of expansion of the anti-cancer T-cells. The antigen polypeptides being larger than the fragments presented by APCs also ensures that a wide variety of epitopes of each antigen will be presented.

The method of the present invention allows for a single tumor neoantigenic construct to be associated with a given particle, or for more than one different neoantigenic construct to be associated with a given particle. Particles associated with the same tumour neoantigen construct or constructs are considered to be the same particle species. Particles associated with different tumour neoantigen construct or constructs are considered to be different particle species.

In general, 100 to 1×10⁹ , for example 100 to 1×10⁸ , for example 100 to 1×10⁷ particles are used in a given expansion run, for example 1000 to 1×10⁷ particles. For example, the ratio of the particle concentration to the APC cell concentration is in the range 1000:1 to 1:10. The ratio can be optimised depending on the size of the particles. For example, for particles of 1µm cross-section, the ratio may be in the range 50:1 to 2:1, for example 25:1 to 5:1, for example 15:1 to 7:1, for example 10:1.

The particles may be a homogenous population, i.e. all of the particles are of the same particle species. Alternatively, the particles may be a heterogeneous population, i.e. the particle population comprises more than one particle species. For example, step a) may additionally comprise: providing a second species of phagocytosable particle, having one or more tumor neoantigenic constructs tightly associated thereto, wherein the tumor neoantigenic constructs comprise an amino-acid sequence comprising at least one mutated amino acid known or suspected to be associated with a cancer in a subject, or a mutated amino-acid sequence known or suspected to be expressed in a cancer cell in the subject, and wherein the sequence of the tumor neoantigenic construct(s) associated with the second particle is different to the sequence of the tumor neoantigenic construct(s) associated with the first particle. Optionally third, and optionally fourth, and optionally 5^{th}, and optionally 6^{th}, and optionally 7^{th}, and optionally 8^{th}, and optionally n^{th} (wherein n may be any integer up to 50) species of phagocytosable particles may be provided, each of them having one or more tumor neoantigenic constructs tightly associated thereto, wherein the tumor neoantigenic construct comprises an amino-acid sequence comprising at least one mutated amino acid known or suspected to be associated with a cancer in a subject, or a mutated amino-acid sequence known or suspected to be expressed in a cancer cell in the subject, and wherein the sequence of the tumor neoantigenic construct(s) associated with the nth species of particle is different to the sequence of the tumor neoantigenic construct(s) associated with the other species of particle.

Thus, in the method of the invention, a first particle associated with a first tumor neoantigenic construct (a particle of a first species) may be used in combination with a second particle associated with a second tumor neoantigenic construct (a particle of a second species), wherein the first and second tumor neoantigenic constructs have different sequences (for example, wherein the first tumor neoantigenic constructs comprise one or more different neoantigen epitopes compared to the second tumor neoantigenic construct; and preferably the first tumor neoantigenic construct comprises neoantigen epitope(s) that are all different compared to the second tumor neoantigenic construct).

Further particle types may also be used, for example 3 or more species of particles (e.g., 3, 4, 5, 6, 7, 8, 9, 10, 11, 12, 13, 14, 15, 16, 18, 19, 20, 25, 30, 40 or 50) may be used. Preferably, 2, 3, 4, 5, 6, 7, 8, 9, 10, 11, or 12 particle species may be used, and more preferably 2, 3, 4, 5, 6, 7, or 8 particle species may be used (for example, 4, 5 or 6 particle types). In embodiments of the method of the invention where more than one particle species is used, each particle species has tumor neoantigenic construct(s) associated with it that have different sequences to the tumor neoantigenic construct(s) associated with the other particle species (for example, each particle species has tumor neoantigenic construct(s) comprising one or more different neoantigen epitopes compared to the tumor neoantigenic construct(s) associated with the other particle species; and preferably each particle species has tumor neoantigenic construct(s) associated with it that have all different neoantigen epitope(s) compared to the tumor neoantigenic construct(s) associated with the other particle species). In embodiments of the method of the invention where more than one particle species is used, the tumor neoantigenic construct on each particle is a construct as described above, comprising one or more neoantigen epitope as described above. Preferably the tumor neoantigenic construct associated with each particle species has two or more covalently linked peptides, and more preferably three or more covalently linked peptides (for example three, four, five, six, seven, eight, nine or ten covalently linked peptides, preferably three, four, five, or six covalently linked peptides, and more preferably three, four or five covalently linked peptides), each peptide comprising an amino-acid sequence comprising at least one mutated amino-acid known or suspected to be associated with a cancer in a subject (and more preferably each being a neoantigen epitope). Preferably, the linked peptides are each linked via a spacer moiety. Each linker moiety may be the same or may be different.

Most preferably, the tumor neoantigenic construct associated with each particle species has three covalently linked peptides, each comprising an amino-acid sequence comprising at least one mutated amino-acid known or suspected to be associated with a cancer in a subject (and more preferably each being a neoantigen epitope). In such embodiments of the method of the invention, preferably 2, 3, 4, 5, 6, 7, 8, 9, 10, 11, or 12 particle species are used, and more preferably 4, 5 or 6, and even more preferably 5.

Degradation of antigens by the APCs is not a uniform process. This ensures that a wide variety of epitopes of each antigen will be presented. Thus, when the tumor neoantigenic constructs are degraded by the APCs, this further increases the variety of epitopes that will be presented. By utilizing the phagocytic route of the APCs, combined with the possibility of each tumor neoantigenic construct having one or more neoantigen epitope, and the possibility of using one or more tumor neoantigenic construct on a particle, improved expansion of T-cells is achieved. That is because the likelihood of providing epitopes for a greater number of anti-tumor T-cells is increased.

A peptide comprising an amino-acid sequence comprising at least one mutated amino-acid known or suspected to be associated with a cancer in a subject of the neoantigenic construct may be a mutated sequence known to be present in the tumor of the subject. For example, the mutation data (both whole exome and hotspot-analyses) or the antigen profile of the cancer type may be known; for example mutation data profiles of certain cancers are included in databases, such as the COSMIC database (accessible at http://cancer.sanger.ac.uk/cosmic). The mutated antigens in the antigen profile can then be targeted, i.e. the one or more peptides in the tumor neoantigenic construct can be designed to have an amino-acid sequence comprising at least one mutated amino-acid sequence in the antigen profile.

A peptide comprising an amino-acid sequence comprising at least one mutated amino-acid known or suspected to be associated with a cancer in a subject of the tumor neoantigenic construct may be a sequence known to be expressed by the tumor or the tissue in which the tumor is present. As such, antigens specific to a particular cancer or to a particular tissue or organ where the cancer is located can be selected. Databases are available, such as EBI expression atlas (https://www.ebi.ac.uk/gxa/home) that provide information on proteins/peptides which are expressed in restricted tissues or organs. Peptides unique to proteins expressed in a particular tissue can then be targeted.

Alternatively (or additionally) the DNA sequence of a cancer cell of the cancer can be established, and thus the mutations in the cancer cell deduced. The mutated sequences can then be targeted, i.e. the one or more peptides in the tumor neoantigenic construct can be designed to have an amino-acid sequence comprising a mutated amino-acid sequence in the cancer cell.

In certain preferred embodiments of the method of the invention, the T-cell sample is from the subject undergoing treatment and the sequence of one or more peptide of each tumor neoantigenic construct is derived from the subject (in particular derived from the tumor antigen profile and/or the DNA sequence of a cancer cell of the subject). Preferably the APC is also from the subject. In another embodiment, the T-cell sample is from the subject undergoing treatment, the APC also from the subject, and the sequence of each peptide of each tumor neoantigenic construct may be derived from a library of mutated sequences for the same species for the cancer which the subject has.

### Antigen-presenting cell (APC) and the T-cell sample

In the context of the present invention, the APC is a professional antigen presenting cell, such as a monocyte/macrophage or a dendritic cell. The APC may be a primary cell or an immortalized cell. Preferably the antigen presenting cell is a phagocyte, more preferably it is a monocyte or dendritic cell.

The APC must be compatible with the T-cells of the T-cell sample, such that they are capable of presenting antigens to the T-cells in an antigen-specific context (MHC restricted) that the T-cells can react to. The APC and the T-cell sample are preferably obtained from the same species and donor-matched with respect to MHC receptors. However, use of genetically engineered APCs from a different species is also envisioned. More preferably the APC and the T-cell sample are obtained from the same subject. If the antigen-presenting cell and the T-cell sample are derived from the same subject, any potential for a mismatch between the APC and the T-cells is avoided.

The antigen-presenting cell and the T-cell sample may be derived from the same blood sample, which is advantageous from a practical point of view. The antigen-presenting cell and the T-cell sample may be derived from a PBMC-sample from the same subject. Obtaining PBMC from peripheral blood samples is a routine protocol, which provides a convenient source for both APCs and T-cells at the same time and from the same subject.

The PBMC sample may be freshly used or subjected to freezing. The possibility of using frozen cells is of great practical advantage from a logistical point of view.

The T-cell sample may be derived from a tumor, preferably a lymphatic vessel in a tumor. Even more preferably the T-cell sample may be derived from the sentinel nodes (i.e. tumor draining lymph nodes).

The T-cell sample may comprise whole PBMCs including both CD4+ and CD8+ T-cells, purified T-cell populations, or PBMCs depleted of (a) particular T-cell population(s). Preferably the T-cell sample comprises both CD4+ and CD8+ T-cells.

Preferably, the anti-tumor T-cells expanded in the method of the invention are CD4+ helper and/or CD8+ T-cells. Anti-tumour CD4+ helper T-cells are able to orchestrate an anti-tumour response. CD8+ T-cells are able to attack the tumour. A mixed T-cell response may thus be beneficial. There is some evidence in the examples described herein that a majority of the anti-tumour T-cells expanded in the method of the invention are CD4+ helper cells.

### Details of the method steps

The present invention provides a method for the expansion of anti-tumor T-cells from a subject with cancer. The method of the invention is carried out *in vitro.* Preferably the subject is a mammal and more preferably, a human. Step (e) preferably lasts from 1 day to 8 weeks, preferably 3 days to 8 weeks, preferably 1 week to 6 weeks, and more preferably from 1 week to 4 weeks. The method of the invention may further comprise before step (a) a step (a'): providing a phagocytosable particle and tightly associating one or more tumor neoantigenic construct to the phagocytosable particle.

In one embodiment of the method of the invention, the particle having one or more tumor neoantigenic constructs tightly associated thereto, the antigen presenting cell and the T-cell sample may be contacted concurrently, in the same container. In another embodiment, the particle having one or more tumor neoantigenic constructs tightly associated thereto, and the antigen presenting cell are first contacted, and the T-cell sample subsequently added. In another embodiment, the antigen presenting cell and the T-cell sample are in a single sample, and the particle having one or more tumor neoantigenic constructs tightly associated thereto is added to the T-cell/antigen presenting cell sample.

In one embodiment of the method of the invention, step (e) of contacting the T-cell sample with the antigen-presenting cell contacted with the particle, further comprises adding low doses IL-2 to the T-cell sample, for example greater than 1.25 U/ml (for example 1.25 U/ml, 2.5 U/ml, 5 U/ml, or 50 U/ml), preferably greater than 2.5 U/ml, 5 U/ml, or 50 U/ml. Antigen specific T-cell expansion occurs in the presence of the antigen-presenting cell when IL-2 is simultaneously present. The IL-2 promotes the differentiation of T-cells into effector T-cells and into memory T-cells. Following expansion of antigen specific T-cells, the antigen-presenting cells may be removed from the expanded T-cell population, for example by magnetic separation.

In another embodiment of the method of the invention, step (e) of contacting the T-cell sample with the antigen-presenting cell contacted with the particle, may further comprise adding IL-2 and/or IL-7 and/or IL-15 to the T-cell sample, for example a low dose of IL-2 to the T-cell sample, for example greater than 1.25 U/ml (for example 1.25 U/ml, 2.5 U/ml, 5 U/ml, or 50 U/ml), preferably greater than 2.5 U/ml, 5 U/ml, or 50 U/ml of of IL-2, with optional addition of IL-7 and/or IL-15.

In one embodiment of the method of the invention, in each of the steps (i.e. in steps (a) to (e), as well as in any further steps, when present) no anti-CD3 antibody and/or anti-CD28 antibody is added during any of the steps. In particular, no anti-CD3 antibody and/or anti-CD28 antibody is added during step (d) or (e) (or, when present, steps (e1), (f), or (g)). In one embodiment of the method of the invention, in each of the steps (i.e. in steps (a) to (e), as well as in any further steps, when present) the conditions for each step are free from anti-CD3 antibody and/or anti-CD28 antibody. In particular, in steps (d) and (e), as well as, when present, steps (e1), (f), or (g) the T-cell sample in each step is free from anti-CD3 antibody and/or anti-CD28 antibody. Anti-CD3 antibody and anti-CD28 antibody can stimulate T-cells indiscriminately, causing expansion of T-cells that are unspecific to the antigen of interest, as well as other T-cells.

In preferred embodiments of the invention, the method does not comprise any step of separating or isolating the antigen specific T-cells during or after step (e) (or step (e1)). More specifically, there is no step of separating or isolating T-cells that have been activated in response to antigen presented by the antigen-presenting cell in the T-cell sample from T-cells in the T-cell sample that have not been activated. That is because the method of the present invention leads to expansion of only the required T-cells which are specific to the antigen that is presented. The T-cells that are not activated in response to antigen presented by the antigen-presenting cell in the T-cell sample in step (e) of the method of the invention will die during the process. As such, no separation or isolation step is required.

### Washes

Preferably the conditions in the step of contacting the T-cell sample with the antigen-presenting cell contacted with the particle having associated peptide are aseptic, and more preferably sterile. The use of the particles allows sterilisation of the particle having the associated peptide, and more especially allows the use of harsh sterilisation conditions, such that all pathogens can be removed, without loss or destruction of the neoantigenic constructs associated with the particles.

As such, in certain preferred embodiments, the method of the invention may comprise before step (a) (and, where applicable, after step (a')): (a") subjecting the tumor neoantigenic construct tightly associated with the particle to a sterilising wash resulting in removal of materials that would be detrimental to the subject. The sterilising wash may also be a denaturing wash.

In certain preferred embodiments, the method of the invention may also comprise before step (a) (and, where applicable, after step (a')): (a‴) subjecting the tumor neoantigenic construct tightly associated with the particle to a denaturing wash resulting in an endotoxin level low enough to not interfere with the subsequent steps (i.e. steps (a) to (e), or when present steps (a) to (f) or (a) to (g)).

In such embodiments comprising step (a") and/or step (a‴), the sterilising wash or denaturing wash may involve subjecting the particle with the associated polypeptide construct(s) to a high pH, for example at least pH 12, preferably at least pH 13, more preferably at least pH 14, and most preferably at least pH 14.3. Alternatively the denaturing wash may involve subjecting the particle with the associated polypeptide construct(s) to a low pH, for example less than pH 3, and more preferably less than pH 2, and most preferably less than pH 1.

In such embodiments comprising step (a") and/or step (a‴), the sterilising wash or denaturing wash may additionally or alternatively involve subjecting the particle with the associated polypeptide construct to a high temperature, such as at least 90°C, preferably at least 92°C, more preferably at least 95°C, for example at least 100°C or at least 110°C. The sterilising wash or denaturing wash may also additionally or alternatively involve subjecting the particle with the associated polypeptide construct to a denaturing agent, such as urea or guanidine hydrochloride at a sufficient concentration, such as at least 5M, 6M, 7M or 8M.

The sterilising wash may involve subjecting the particle and associated polypeptide construct to alkali, preferably a strong alkali, for example at least 0.1M, 0.5M, 1M, 2M, 3M, 4M, 5M, 6M, 7M or 8M alkali. In certain preferred embodiments, the sterilising wash may involve subjecting the particle and associated polypeptide construct to at least 1M sodium hydroxide (NaOH), preferably at least 2M NaOH. Other alkalis that may be used include, but are not limited to: Lithium hydroxide (LiOH), Potassium hydroxide, (KOH), Rubidium hydroxide (RbOH), Cesium hydroxide (CsOH), Magnesium hydroxide (Mg(OH)₂), Calcium hydroxide (Ca(OH)₂), Strontium hydroxide (Sr(OH)₂), and Barium hydroxide (Ba(OH)₂).

The sterilising wash may involve subjecting the particle and associated polypeptide construct to acid, preferably a strong acid, for example at least 0.1M, 0.5M, 1M, 2M, 3M, 4M, 5M, 6M, 7M or 8M acid. In certain preferred embodiments, the sterilising wash may involve subjecting the particle and associated polypeptide construct to at least 1M hydrochloric acid (HCl), preferably at least 2M HCl. Other acids that may be used include, but are not limited to: hydroiodic acid (HI), hydrobromic acid (HBr), perchloric acid (HClO₄), nitric acid (HNO₃) and sulfuric acid (H₂SO₄).

In preferred embodiments, the sterilisation wash results in the particles and associated polypeptide constructs being aseptic, and more preferably the particles and associated polypeptide constructs being sterile. Aspetic as defined herein as being free from disease-causing bacteria or other living microorganisms. Sterile is defined herein as free from bacteria or other living microorganisms.

In preferred embodiments, the denaturing wash results in an endotoxin amount such that in the final concentration of endotoxin is less than 100 pg/ml, preferably less than 50 pg/ml, more preferably less than 25 pg/ml and most preferably less than 10 pg/ml.

If the preparation is contaminated with viable microorganisms, the subsequent steps in cell culture are likely to be compromised. As such, in preferred methods of the invention the particle with the associated polypeptide construct of step (a) has been subjected to a sterilising wash resulting in a micro-organism and endotoxin level low enough to not interfere with the subsequent steps.

The particular manner of sterilising wash is not critical in the context of the present invention. For instance, the sterilising wash may involve subjecting the particle and associated polypeptide construct to a high pH, to a low pH, to a high temperature, to a steralising/denaturing agent or a combination thereof. Preferably, the sterilising wash involves subjecting the particle and associated polypeptide construct to a high pH or to a strong steralising/denaturing agent such as 8M urea or 6M guanidine-HCl. Most preferably, the sterilising wash involves subjecting the particle and associated polypeptide construct to a high pH of at least 13.0, more preferably at least 14.0, most preferably at least 14.3. Preferably, the denaturing may involve subjecting the particle and associated polypeptide construct to a wash with 1-5M, preferably 1-3M, more preferably 1.5-2.5M, most preferably 2M strong alkali, such as NaOH or KOH, preferably NaOH.

A particular advantage with a sterilising wash is that the conditions may be selected such that the preparation with the particles and the associated polypeptide construct is both sterilized and denatured in a single step. In particular, high pH wash (e.g. pH >14) can conveniently simultaneously and quickly sterilize the preparation and achieve a denaturing wash effective in eliminating endotoxins.

The sterilisation wash may comprise a single wash or several repeated washes, such as 2, 3, 4 or 5 washes. The denaturing wash may comprise a single wash or several repeated washes, such as 2, 3, 4 or 5 washes.

### Determining the degree of T-cell activation

After step (e) the method of the invention may further comprise a step of determining the degree of anti-tumor T-cell activation in the T-cell sample, for example by comparing the degree of anti-tumor T-cell activation to a relevant reference, whereby a higher degree of anti-tumor T-cell activation in the sample compared to the reference is indicative of the conclusion that the one or more tumor neoantigenic determinant results in anti-tumor T-cell activation in the sample. Determining the degree of anti-tumor T-cell activation in the T-cell sample may involve determining the fraction of activated T-cells in the sample. Determining the degree of anti-tumor T-cell activation in the T-cell sample may be performed using an ELISpot or a FluoroSpot-technique.

The method of the invention may comprise the step of comparing the degree of T-cell activation to a relevant reference, whereby a higher degree of T-cell activation in the sample compared to the reference leads to the conclusion that the candidate antigen results in antigen-specific T-cell activation in the sample. The reference is critical for defining "activation", so degree of activation is preferably defined in comparison to reference samples. The reference samples may, for example, be samples from normal tissue when analysing tumor or tumor draining lymph node samples. The reference samples are used to set the threshold for diagnostic/prognostic conclusion, and determine antigen specific positive i.e. activation, or negative i.e. downregulation.

Determining the degree of T-cell activation in the T-cell sample may involve determining the fraction of activated T-cells in the sample, i.e. the number of activated T-cells in relation to the total T-cells in the sample. The fraction of activated cells in relation to the fraction of activated cells in the reference sample will provide a measure of the magnitude of T-cell activation. Preferably, in each analysis the level of spontaneous activation ("background activation level") in individual samples is determined in samples incubated without antigen, i.e. negative control. The background activation may be compensated for in the analysis; in other words, fractions of activation may be calculated on net values where the fraction of activation to negative control is subtracted.

Several methods suitable for determining antigen-specific T-cell activation exist, including ELISpot, Fluorospot, intracellular staining of cytokines with flow cytometry, FASCIA, proliferation assays (eg thymidine incorporation, CFSE or BrdU staining), specific TCR-detection with MHC-I or II tetramers, and ELISA- or Luminex analysis of secreted cytokines.

With the ELISpot technique, one directly detects release of a specific cytokine by cells in response to a stimulus. The cells are seeded on a membrane and the number of cytokine secreting cells is measured. Depending on which cytokine is being measured, different variables such as macrophage or T-cell activation can be measured. FluoroSpot builds upon the ELISpot technique and allows for simultaneous readings of several different secreted cytokines. This allows for a more exact and nuanced estimation of cell activation. Both of these methods offer a quick and easy way of measuring T-cell activation. Preferably, determining the degree of T-cell activation in the T-cell sample may be performed using an ELISpot/FluoroSpot-technique or a proliferation assay (i.e thymidine incorporation). Determining the degree of T-cell activation in the T-cell sample may comprise determining the fraction of T-cells contacted with the antigen-presenting cell responding by secretion of Interferon gamma (IFN-y), Interleukin 17 (IL-17), Interleukin 22 (IL-22) or a combination thereof, e.g. by means of an ELISpot or FluoroSpot assay. Other relevant analytes (combination(s) of cytokines) can be used for analysing specific diseases or conditions, such as T-reg cytokines.

### Optional further activation step

In certain embodiments of the method of the invention, the method further comprises, after step (e) (for example immediately after step (e), or immediately after step (f), or immediately after step (g); preferably immediately after step (e) or immediately after step (f)):
e1) contacting the T-cell sample with a second antigen-presenting cell contacted with a second particle *in vitro* under conditions allowing specific activation of anti-tumor T-cells in response to antigen presented by the antigen-presenting cell.

Step (e1) advantageously acts as a second round of activation and stimulation ("re-stimulation") of the T-cells. Thus step (e1) may be referred to as a "re-stimulation" step. Step (e1) may, for example, last from 1 to 4 weeks, and more preferably from 2 to 3 weeks.

Preferably, in certain embodiments of the method of the invention, the method further comprises, after step (e) (for example immediately after step (e), or immediately after step (f), or immediately after step (g); preferably immediately after step (e) or immediately after step (f)):
a1) providing a second phagocytosable particle, having one or more tumor neoantigenic constructs tightly associated thereto, wherein the tumor neoantigenic construct comprises an amino-acid sequence comprising at least one mutated amino acid known or suspected to be associated with a cancer in a subject, or a mutated amino-acid sequence known or suspected to be expressed in a cancer cell in the subject;
b1) providing a second viable antigen-presenting cell;
c1) contacting the second particle with the second antigen-presenting cell *in vitro* under conditions allowing phagocytosis of the particle by the antigen-presenting cell; and
e1) contacting the T-cell sample with the second antigen-presenting cell contacted with the second particle *in vitro* under conditions allowing specific activation of anti-tumor T-cells in response to antigen presented by the antigen-presenting cell.

Step (e1) (or, when present, steps (a1) to (e1)) are preferably performed after step (e) and/or (f) and/or (g) when expansion rate of the T-cell sample wanes. Preferably step (e) lasts from 2 to 4 weeks (which is when expansion rate of the T-cell sample during step (e) is expected to wane), and step (e1) (or, when present, steps (a1) to (e1)) are performed after step (e).

In embodiments where step (e1) is after step (f), the method may further comprise (f1): determining the degree of anti-tumor T-cell activation in the T-cell sample, for example by comparing the degree of anti-tumor T-cell activation to a relevant reference, whereby a higher degree of anti-tumor T-cell activation in the sample compared to the reference is indicative of the conclusion that the one or more tumor neoantigenic determinant results in anti-tumor T-cell activation in the sample.

In embodiments where step (e1) is after step (g), the method may further comprise (g1): removing the particles and antigen-presenting cells from the expanded T-cell sample.

The second phagocytosable particle having one or more tumor neoantigenic constructs tightly associated thereto may be the same as the phagocytosable particle, having one or more tumor neoantigenic constructs tightly associated thereto of step (a), or it may be different. Preferably it is the same.

The second viable antigen-presenting cell may be the same as the viable antigen-presenting cell of step (b), or it may be different. Preferably it is the same.

### Removal of particles

After step (e) (and where applicable, after step (e1)), the method of the invention may further comprise step (f) removing the particle from the T-cell sample; and/or (g) removing the particle and antigen-presenting cell from the T-cell sample.

The particles and any antigen-presenting cells having internalised particles can advantageously be completely removed from the T-cell sample using a magnet or magnetic field, for example before transfusing the expanded T-cell sample back into the subject. As such, the T-cell sample at the end of the method of the invention comprises only the expanded T-cells and any antigen-presenting cell not having an engulfed particle. Therefore, in certain preferred embodiments of the method of the invention, step (f) comprises removing the particle from the T-cell sample by means of a magnet or a magnetic field. Removal of the particles in step (f) also removes antigen-presenting cells having an internalised particle. Typically, the antigen-presenting cells having an internalised particle will be dead when the particles (and the antigen-presenting cells having an internalised particle) are removed in step (f) or (g).

### Treatments

The method of the invention provides anti-tumor T-cells. The anti-tumor T-cells are useful in the treatment of cancer in the subject. The T-cells are the cells obtained after step (f) or (g) of the method of the invention. The expanded T-cells may be administered intravenously, intraarterially, intrathecally or intraperitoneally.

The cancer may be any form of solid cancer. A solid cancer in the context of the present invention is an abnormal mass of tissue that originates in an organ. A solid cancer usually does not contain cysts or liquid areas. The solid cancer may be malignant. Different types of solid cancers are named for the type of cells that form them. Types of solid cancer include sarcomas, carcinomas, and lymphomas.

The present invention provides a composition comprising anti-tumor T-cells produced by a method comprising the steps of:
a) providing a phagocytosable particle, the particle having paramagnetic properties and having one or more tumor neoantigenic constructs tightly associated thereto, wherein the tumor neoantigenic construct comprises an amino-acid sequence comprising at least one mutated amino acid knownor suspected to be associated with the cancer in the subject, or a mutated amino-acid sequence known or suspected to be expressed in a cancer cell in the subject;
b) providing a viable antigen-presenting cell;
c) contacting the particle with the antigen-presenting cell *in vitro* under conditions allowing phagocytosis of the particle by the antigen-presenting cell;
d) providing a T-cell sample comprising viable T-cells from the subject;
e) contacting the T-cell sample with the antigen-presenting cell contacted with the particle *in vitro* under conditions allowing specific activation and expansion of anti-tumor T-cells in response to antigen presented by the antigen-presenting cell;
(f) removing the particle from the T-cell sample and/or removing the particle and antigen-presenting cell from the T-cell sample;
wherein the T-cell sample in steps (d) to (f) is free from anti-CD3 and/or anti-CD28 antibodies.

The T-cell composition of the invention comprises a mixture of CD4+ helper cells and CD8+ T-cells. For example, in one embodiment, the mixture may comprise predominantly CD4+ helper cells and also CD8+ T-cells.

The present invention also provides a composition comprising anti-tumor T-cells produced by the method described above for use in the treatment of cancer in a subject.

Examples of solid cancers include adrenal cancer, anal cancer, anaplastic large cell lymphoma, angioimmunoblastic T-cell lymphoma, B-cell lymphoma, bile duct cancer, urinary bladder cancer, brain/CNS tumors, breast cancer, cervical cancer, colon cancer, endometrial cancer, esophagus cancer, ewing family of tumors, eye cancer, gallbladder cancer, gastrointestinal carcinoid tumors, gastrointestinal stromal tumor (gist), gestational trophoblastic disease, hepatosplenic T-cell lymphoma, Hodgkin's lymphoma, intravascular large B-cell lymphoma, kidney cancer, laryngeal and hypopharyngeal cancer, liver cancer, lung cancer (non-small cell and small cell), lung carcinoid tumor lymphomatoid granulomatosis, malignant mesothelioma, nasal cavity and paranasal sinus cancer, nasopharyngeal cancer, neuroblastoma, nodal marginal zone B cell lymphoma, non-Hodgkin's lymphoma, oral cavity and oropharyngeal cancer, osteosarcoma, ovarian cancer, pancreatic cancer, penile cancer, pituitary tumors, primary effusion lymphoma, prostate cancer, retinoblastoma, rhabdomyosarcoma, salivary gland cancer, sarcoma, skin cancer (basal and squamous cell, melanoma and merkel cell), small intestine cancer, stomach cancer, testicular cancer, thymus cancer, thyroid cancer, uterine sarcoma, vaginal cancer, vulvar cancer, Waldenstrom macroglobulinemia, and Wilms' tumor. The T-cell composition of the invention is especially effective in the treatment of solid cancers. As such, the subject of the invention may have a solid cancer. The T-cell composition of the invention is particularly effective in the treatment of solid cancers selected from the group consisting of: anal cancer, urinary bladder cancer, breast cancer, cervical cancer, colon cancer, liver cancer, lung cancer (non-small cell and small cell), lung carcinoid tumor, ovarian cancer, pancreatic cancer, penile cancer, prostate cancer, stomach cancer, testicular cancer, uterine sarcoma, vaginal cancer, vulvar cancer, and even more especially for the treatment of breast cancer, colon cancer, liver cancer, lung cancer (non-small cell and small cell), lung carcinoid tumor, pancreatic cancer, prostate cancer, ovarian cancer and urinary bladder cancer.

The T-cell composition of the invention are also especially effective in the treatment of metastatic solid cancers. Metastatic cancer is cancer which has spread from the primary site of origin into one or more different areas of the body.

The cancer may alternatively be any form of hematologic malignancy. A hematologic malignancy in the context of the present invention is a form of cancer that begin in the cells of blood-forming tissue, such as the bone marrow, or lymphatic system. In many hematologic malignancies, the normal blood cell development process is interrupted by uncontrolled growth of an abnormal type of blood cell. Examples of hematologic cancer include leukemias, lymphomas, myelomas and myelodysplastic syndromes (lymphomas may be classed as both a solid cancer and a hematologic malignancies).

Examples of hematologic malignancies include acute basophilic leukemia, acute eosinophilic leukemia, acute erythroid leukemia, acute lymphoblastic leukemia, acute megakaryoblastic leukemia, acute monocytic leukemia, acute myeloblastic leukemia with maturation, acute myelogenous leukemia, acute myeloid dendritic cell leukemia, acute promyelocytic leukemia, adult T-cell leukemia/lymphoma, aggressive NK-cell leukemia, anaplastic large cell lymphoma, and plasmacytoma, angioimmunoblastic T-cell lymphoma, B-cell chronic lymphocytic leukemia, B-cell leukemia, B-cell lymphoma, B-cell prolymphocytic leukemia, chronic idiopathic myelofibrosis, chronic lymphocytic leukemia, chronic myelogenous leukemia, chronic myelomonocytic leukemia, chronic neutrophilic leukemia, extramedullary, hairy cell leukemia, hepatosplenic T-cell lymphoma, Hodgkin's lymphoma, intravascular large B-cell lymphoma, Kahler's disease, lymphomatoid granulomatosis, mast cell leukemia, multiple myeloma, myelomatosis, nodal marginal zone B cell lymphoma, non-Hodgkin's lymphoma, plasma cell leukemia, primary effusion lymphoma, and Waldenstrom macroglobulinemia.

In one preferred embodiment, when the T-cell composition of the invention is used in the treatment of cancer in a subject, said subject has not been administered any chemotherapy to lower the number of immune cells in the body within 1 week before (preferably within 4 weeks before) the administration of the T-cell composition of the invention.

An advantage of the present invention is that repeat treatment with the anti-tumor T-cell composition of the invention is only required if memory T-cells that recognise the neoantigen(s) become depleted, or if the cancer evolves so that different neoantigens are present on the tumor from the original neoantigen(s) targeted by the T-cells. If that is the case, and the cancer becomes resistant to the initial T-cell treatment, the treatment can be carried out a second time by administering a further T-cell composition of the invention, wherein in step (a) the phagocytosable particles have different tumor neoantigenic constructs tightly associated thereto compared to the tumor neoantigenic construct(s) used to produce the T-cell of the previous treatment. For example, the neoantigen construct has a different sequence, e.g. comprising one or more different neoantigen epitopes compared to the tumor neoantigenic constructs tightly associated with the phagocytosable particle used in the previous method to make the a first anti-tumor T-cell composition; or the neoantigen construct comprises all different neoantigen epitopes compared to the tumor neoantigenic constructs tightly associated with the phagocytosable particle used in the previous method to make the a first anti-tumor T-cell composition. For example the neoantigen epitopes in the second treatment are epitopes that were not present in the first treatment, for example because they had yet developed in the tumour at that time.

The present invention also provides a composition comprising anti-tumor T-cells produced as described above for use in the treatment of cancer in a subject, wherein after administration of the composition comprising anti-tumor T-cells to the subject, the subject is monitored for the presence of neoantigen specific T-cells (for example memory T-cells) derived from the anti-tumor T-cells produced as described above at X days after administration of the composition, wherein X is around 7, 14, 21, 28, 30, 35, 42, 49, 56, 60, 90, 120, 150, 180, 300, and/or 365 days; and optionally the subject is administered further anti-tumor T-cells produced as described above.

The present invention also provides a composition comprising anti-tumor T-cells produced as described above for use in the treatment of relapsing cancer in a subject who has been previously treated with a first anti-tumor T-cell composition produced according to the invention, wherein the composition comprising anti-tumor T-cells is produced as described above wherein in step (a) the tumor neoantigenic constructs tightly associated with the phagocytosable particle has a different sequence to the tumor neoantigenic constructs tightly associated with the phagocytosable particle used in the method to make the a first anti-tumor T-cell composition produced as described above. For example, in step (a) the tumor neoantigenic constructs tightly associated with the phagocytosable particle comprises one or more different neoantigen epitopes compared to the tumor neoantigenic constructs tightly associated with the phagocytosable particle used in the method to make the a first anti-tumor T-cell composition produced as described above; and preferably in step (a) the tumor neoantigenic constructs tightly associated with the phagocytosable particle comprises all different neoantigen epitopes compared to the tumor neoantigenic constructs tightly associated with the phagocytosable particle used in the method to make the a first anti-tumor T-cell composition produced as described above.

### Particles:

Disclosed herein (but not claimed) is a paramagnetic or superparamagnetic phagocytosable particle, having one or more tumor neoantigenic constructs tightly associated thereto, wherein the tumor neoantigenic construct comprises an amino-acid sequence comprising at least one mutated amino-acid known or suspected to be associated with a cancer in a subject.

Preferably, the particles may have a largest dimension of less than 5.6 µm, preferably less than 4 µm, more preferably less than 3 µm, for example less than 2.5 µm, less than 2 µm or less than 1.5 µm. The particles may be used in a method of the invention and preferably may have a largest dimension of greater than 0.001 µm, preferably greater than 0.005 µm, preferably greater than 0.01 µm, preferably greater than 0.05 µm, preferably greater than 0.1 µm, more preferably greater than 0.2 µm, and even more preferably greater than 0.5 µm. Preferably, the particles may have a largest dimension in the interval 0.1-5.6 µm, preferably 0.2-5.6 µm, preferably 0.5-5.6 µm, preferably 0.1-4 µm, preferably 0.5-4 µm, more preferably 0.1-3 µm, more preferably 0.5-3 µm, even more preferably 0.1-2.5 µm, even more preferably 0.5-2.5 µm, even more preferably 0.2-2 µm, even more preferably 0.5-2 µm, more preferably 1-2 µm, or for example about 1 µm, about 1.5 µm or about 2 µm (preferably about 1 µm). Preferably, the particle is substantially spherical.

Preferably, the tumor neoantigenic construct is covalently attached to the particle. For example the polypeptide may be attached by an amide bond between an amine group or a carboxylic acid group of the polypeptide, and a carboxylic acid group or an amine group on the surface of the particle.

Alternatively, the candidate antigen polypeptide may be linked to the particles via a metal chelate.

For example, particles linked with a metal chelating ligand, such as iminodiacetic acid can bind metal ions such as Cu²⁺, Zn²⁺, Ca²⁺, Co²⁺ or Fe³⁺. These metal chelates can in turn bind proteins and peptides containing for example histidine or cystein with great strength. Thus, particles with metal chelates can non-covalently adsorb peptides/proteins, in a manner which allows stringent washing to reduce the amount of LPS and other contaminating components in the bound peptides/proteins.

The particle may comprise polymer, glass or ceramic material (e.g. the particle may be a polymer particle, a glass particle or a ceramic particle). Preferably the particle comprises a synthetic aromatic polymer, such as polystyrene, or, another polymer, such as polyethylene. The particle may comprise polysaccharide polymer, for example agarose. Preferably, the particle does not comprise polysaccharide polymer (and preferably is not a polysaccharide polymer particle). Preferably the particle is a polystyrene or polyethylene particle, and more preferably is a polystyrene particle. Preferably the particle is a monodispersed particle (i.e. a particle with a uniform size).

### Kits

Also disclosed herein (but not claimed) is a kit comprising a paramagnetic or superparamagnetic phagocytosable particle (i.e. a paramagnetic or superparamagnetic phagocytosable particle, having one or more tumor neoantigenic constructs tightly associated thereto, wherein the tumor neoantigenic construct comprises an amino-acid sequence comprising at least one mutated amino-acid known or suspected to be associated with a cancer in a subject) and reagents suitable for use in expanding a T-cell population. The kit may further comprise reagents for assisting in expanding a T-cell population, such as IL-2. It may optionally also comprise IL-7 and/or IL-15. The kit may further comprise reagents for sterilising the particle.

Preferably, the kit does not comprise anti-CD3 antibody and/or anti-CD28 antibody.

Also disclosed herein (but not claimed) is a kit comprising a paramagnetic or superparamagnetic phagocytosable particle, coupling reagents for coupling a peptide to the phagocytosable particle, and reagents suitable for use in expanding a T-cell population.

The kit may further comprise instructions for designing tumor neoantigenic constructs. The kit may further comprise reagents for producing tumor neoantigenic constructs, for example ready-to-use vectors adjusted for different cloning and expression conditions. The kit may also or alternatively comprise one or more tumor neoantigenic constructs, for example it may comprise a library of tumor neoantigenic constructs for one or more specific cancers. The kit may comprise one or more neoantigen epitopes. For example it may comprise a library of neoantigen epitopes for one or more specific cancers. The kit may comprise instructions and/or reagents for producing tumor neoantigenic constructs from the neoantigen epitopes (and optionally one or more spacer moieties).

The kit may further comprise reagents for assisting in expanding a T-cell population, such as IL-2. It may optionally also comprise IL-7 and/or IL-15. The kit may further comprise reagents for sterilising the particle.

Preferably, the kit does not comprise anti-CD3 antibody and/or anti-CD28 antibody. Preferably, the kit includes instructions to not add anti-CD3 antibody and/or anti-CD28 antibody when the kit is used, for example used in a method of the invention. The kit may also comprise instructions not to separate the activated T-cells in the T-cell sample before expanding the T-cells.

### EXAMPLES

### Example 1: Polypeptide coupling to particles

Polypeptides were covalently coupled to paramagnetic particles containing free carboxylic acid groups. Dynabeads^{®} MyOne^{™} Carboxylic Acid (ThermoFischer Scientific) were used (1 µm diameter spheres) as the particles. The coupling procedure was carried out according to the manufacturers protocol (Two-Step procedure using NHS (N-Hydroxysuccinimide) and EDC (ethyl carbodiimide)):
Particles were washed twice with MES-Buffer (25mM MES (2-(N-morpholino)ethanesulfonic acid), pH 6). The carboxylic acid groups were then activated by adding 50 mg/ml NHS (N-Hydroxysuccinimide) and 50mg/ml EDC (N-(3-Dimethylaminopropyl)-N'-ethylcarbodiimide) in MES-buffer to the particles and incubated for 30 minutes in room temperature. The particles were collected with a magnet and the supernatant was removed and the particles washed twice with MES-buffer. The protein was diluted in MES-buffer to a concentration of 1 mg/ml, total 100 ug and added to the particles and incubated for 1h in room temperature. The particles were collected with a magnet and the supernatant was removed and saved for protein-concentration measurement. The non-reacted activated carboxylic acid groups were quenched with 50 mM Tris pH 7,4 for 15 minutes. The particles were then washed with PBS pH 7.4 and then stored in -80°C.

To measure the amount of protein coupled to the particles, a BCA protein assay kit (Pierce BCA Protein Assay Kit, ThermoFisher Scientific) was used to measure the protein concentration of the protein before coupling as well as in the supernatant after coupling. The BCA-assay was used according to the manufacturer's protocol.

The bicinchoninic acid, BCA, protein assay was used to measure protein concentration before and after coupling to ensure successful coupling. Several polypeptides were tested and an average of 48.7 µg (mean: 48.7, SD: 20.5, N=10) was coupled per 1 mg particles. According to manufacturer's instruction, 50 µg polypeptide can be coupled per 1 mg particles, indicating that the efficiency of the coupling done in this example was high.

### Example 2: washes

Particles were coupled with a recombinant protein produced in E. coli. The particles were washed with one of 3 different wash-buffers, 2M NaOH pH 14.3, 8 M Urea or 6 M Guanidine (Guanidine-HCl), all in sterile water at RT, or they were incubated in PBS at 95°C. The particles were suspended in the buffer and shaken for 4 min, collected with a magnet and the supernatant removed. This was repeated 3 times. The heat treated particles were put in PBS pH 7.4 and put in a heating block at 95°C for 5 minutes, then collected with a magnet and the supernatant removed. This was repeated 3 times. The particles were then washed 3 times with sterile PBS to remove any remaining wash-buffer.

Four different washing conditions were tested: (a) High pH (2M NaOH pH 14.3), (b) Heat (95°C) and sterilising/denaturing agents ((c) 8M Urea and (d) 6M guanidine hydrochloride).

After washing, the polypeptide associated with the particles remained associated with the particles.

### Example 3a: Identification of suitable particle size for phagocytosable particles

Proliferation assay with Thymidine incorporation was used to test the effect of particle size on antigen-specific T-cell activation. Splenocytes from ovalbumin (OVA) immunized mice were stimulated with OVA coupled particles of different sizes to measure antigen specific proliferation.

Paramagnetic particles with a diameter of 5.6µm, 1µm and 0.2 µm with carboxylic acid on their surface were coupled with ovalbumin or bovine serum albumin according to the protocol in Example 1.

To test the effectiveness of the particles to stimulate antigen specific T-cell activation a proliferation assay (with 3H thymidine incorporation) was used. Particle concentration in relation to cell concentration was 1:1 for the 5.6µm particles, 10:1 for the 1µm particles and 500:1 for the 0.2µm particles. Total protein concentration during the incubation with the cells was calculated to 125ng/ml, 160ng/ml and 160ng/ml for the 5.6µm, 1µm and 0.2µm respectively. The proliferation assay was run as follows:
Proliferation assay with thymidine incorporation with splenocytes from ovalbumin sensitized mice. As stimuli, ovalbumin (SigmaAldrich) and BSA (SigmaAldrich) coupled to beads (Dynabeads^{®} MyOne^{™} Carboxylic Acid) were used. Mice were immunized to ovalbumin via monthly injections of 100µg ovalbumin (Sigma) adsorbed to aluminium hydroxide. Three months after the first injection the mice were killed and spleens harvested. Splenocytes were prepared by standard procedures, as described in Thunberg et al. 2009, Allergy 64:919.

The cells were incubated in cRPMI either with ovalbumin coupled beads or BSA coupled beads (10 beads per cell) for 5 days. All cells were incubated for 6 days in a humidified atmosphere with 6 % CO2 at 37°C. One ₃Ci/well [3H] thymidine was added to cell cultures for the final 18 hours of incubation. Mean counts per minute (cpm) obtained from stimulated triplicates were divided by mean cpm values from un-stimulated cells and expressed as stimulation indices (SI). SI-values ≥2.0 are generally considered positive.

As seen in Figure 1, cells incubated with OVA-particles with a diameter of 0.2 µm showed increase in proliferation with a mean SI of 4.1 (95%CI 2.4-5.8, P=0.007). The cells incubated with OVA-particles with a diameter of 1µm showed increase in proliferation with a mean SI of 8.4 (95%CI 6.1-10.6, P<0.005). The cells incubated with OVA-particles with a diameter of 5.6 µm failed to stimulate proliferation, mean SI 1.1 (95%CI 0.4-2.7, P=0.876).

These results show that antigen coupled to particles of different sizes can stimulate cell proliferation. The particles with a diameter of about 1 µm seems to be most efficient in regards to cell stimulation but particles down to a size of 0.2 µm still works. It is reasonable to predict that particles of sizes larger than 1 µm also work, although as the diameter comes close to 5.6 µm the particles completely fail to stimulate the cells. It is reasonable to assume that 1 µm is an optimal size, since it is similar to the size of bacteria. Our immune system has evolved to phagocytose and react to microorganisms/particles of this size. A normal antigen presenting cell has a size in the range 10-15 µm.

### Example 3b: Comparison of antigen coupled particles of different particle sizes and their effectiveness in activating and expanding T-cells

### (i) Preparation of antigen coupled phagocytosable particles:

Three kinds of paramagnetic polystyrene phagocytosable particles of different sizes were used:
- diameter of 1µm (Dynabeads MyOne Carboxylic Acid, ThermoFisher),
- diameter of 2.8µm (Dynabeads M-270 Carboxylic acid, ThermoFisher) and
- diameter of 4.5µm (Dynabeads M-450 Epoxy, ThermoFisher).

The phagocytosable particles were coupled with antigen (Cytomegalovirus protein PP65) according to the manufacturer's instruction. To remove endotoxin, all phagocytosable particles were washed five times with a 0,75M sodium hydroxide buffer and subsequently resuspended in sterile PBS.

### (ii) Incubation of antigen coupled phagocytosable particles:

Peripheral blood mononuclear cells (PBMCs) from a healthy donor, isolated via standard ficoll-based density gradient centrifugation were cultured together with the phagocytosable particles coupled with antigen (hereinafter will be referred to as "antigen coupled particles") in a 48-well plate for 18 h at 37°C, 5% CO₂, 500 000 cells/well at a concentration of 1000 000 cells/ml. The concentration of antigen coupled particles were equalized based on total surface area (a surrogate marker for antigen amount as it is bound to the surface of the antigen coupled particles). This equalled to 10 antigen coupled particles/PBMC for the 1µm particles, 1.4 antigen coupled particles/PBMC for the 2.8µm particles and 0.5 antigen coupled particles/PBMC for the 4.5µm particles, based on the number of total PMBCs in the sample.

**Table 1**

| Phagocytosable particle size | Number of PBMCs in sample | Ratio of antigen coupled particles to PBMCs | Number of antigen coupled particles in sample |
|---|---|---|---|
| 1µm | 500 000 | 10:1 | 5 000 000 |
| 2.8µm | 500 000 | 1.4:1 | 700 000 |
| 4.5µm | 500 000 | 0.5:1 | 250 000 |

### (iii) Assessment of uptake:

After incubation, the number of phagocytosed antigen coupled particles were manually counted using a confocal microscope. Eight cells were counted to obtain mean and standard deviation values. In Figure 5A, there are shown images from the confocal microscope of representative cells with intracellular phagocytosed antigen coupled particles. Black dashed line indicates the outline of the cell. The white line shows the dimensions of the total intracellular antigen coupled particles.

This method was not applicable for the 1µm antigen coupled particles as they were too small to accurately count. In order to assess the amount of 1µm antigen coupled particles, the total volume of all phagocytosed antigen coupled particles were measured and the amount of individual antigen coupled particles were back-calculated based on the total volume, assuming a packing density of 60%. This method proved reasonably accurate for the 2.8µm antigen coupled particles (manually counted 9.1 antigen coupled particles/cell vs estimated 11.9 antigen coupled particles/cell) and 4.5µm antigen coupled particles (manually counted 3.1 antigen coupled particles/cell vs estimated 2.4 antigen coupled particles/cell) and can as such be assumed to accurately estimate the amount of 1µm antigen coupled particles as well.

The uptake of antigen coupled particles in shown in Figures 5B and 5C. Figure 5B shows the number of antigen coupled particles taken up by each cell as assessed by manual counting (8 cells counted per bead-type). Using the manual counting method, it was found that the number of phagocytosed antigen coupled particles per cell for the 4.5µm antigen coupled particles was of 3.1 ( ±1.1). For the 2.8µm antigen coupled particles it was 9.1 ( ±2.2). It was not possible to count the number of 1µm antigen coupled particles using this method.

Figure 5C shows the number of antigen coupled particles taken up by each cell as assessed by the volume calculation (3 cells measured per bead-type) (*p<0.05 **p<0.01 ***p<0.001, calculated using Students T-test). Using the volume calculation method, it was found that the number of phagocytosed antigen coupled particles per cell for the 4.5µm antigen coupled particles was of 2.4 ( ±1.1). For the 2.8µm antigen coupled particles it was 11.9 ( ±3.2). For the 1µm antigen coupled particles it was 203.7 ( ±21.9).

Based on the number of antigen coupled particles taken up by each cell as assessed by the volume calculation method, the total phagocytized surface area, and by extension the total amount of antigen, was calculated. The surface area that was taken up was calculated as 639.6 ( ±68.9) µm² for the 1µm antigen coupled particles, 293.1 ( ±79.3) µm² for the 2.8µm antigen coupled particles and 150.7 ( ±67.0) µm² for the 4.5µm antigen coupled particles. These data are shown in Table 2 below.

**Table 2:**

| Phagocytosable particle size | Antigen coupled particles uptake per cell (counted) | Antigen coupled particles uptake per cell (calculated from volume) | Surface area taken up per cell |
|---|---|---|---|
| 1µm | - | 203.7 (±21.9) | 639.6 (±68.9) µm² |
| 2,8µm | 9.1 (±2.2) | 11.9 (±3.2) | 293.1 (±79.3) µm² |
| 4,5µm | 3.1 (±1.1) | 2.4 (±1.1) | 150.7 (±67.0) µm² |

### (iv) Assessment of T-cell stimulation

The ability of the antigen coupled particles to stimulate T-cells and hence promote their expansion was assessed by measuring the release of IFNy, IL22 and IL17A from PBMCs using a FluoroSpot assay (Mabtech, Sweden). PBMCs (250,000/well) from CMV-sensitive healthy donors (n=2) were stimulated with the antigen coupled particles in triplicates. The concentration of antigen coupled particles were as previously described equalized based on total surface area: 10 1µm antigen coupled particles/cell, 1.4 2.8µm antigen coupled particles/cell and 0.5 4.5µm antigen coupled particles/cell.

**Table 3:**

| Phagocytosable particle size | Number of PBMCs per well | Number of monocytes per well |
|---|---|---|
| 1µm | 250,000 | 50,000 |
| 2,8µm | 250,000 | 50,000 |
| 4,5µm | 250,000 | 50,000 |

The cells were incubated for 44h at 37°C, 5% CO₂. The plates were developed according to the manufacturer's instructions and read in an automated FluoroSpot reader. The data reported for the FluoroSpot is spot-numbers when the cells are stimulated with antigen coupled particles above the spot-numbers when not stimulated with antigen coupled particles.

The level of IFNy-production, as assessed in the FluoroSpot assay, is shown in Figure 6A. It is seen that there is little difference between the antigen particles.

The level of IL22 and IL17 production, as assessed in the FluoroSpot assay, is shown in Figures 6B and 6C. It is seen that the 1µm antigen coupled particles caused a significantly higher IL22 and IL17 production in one individual than the larger antigen coupled particles, with a similar trend seen for the other individual in regards to IL22.

The level of dual-cytokine production, as assessed in the FluoroSpot assay, is shown in Figures 6D and 6E. It is seen that the 1µm antigen coupled particles caused a significantly higher dual-cytokine release (IFNγ+IL17 and IL22γ+IL17) for one healthy donor when stimulated with the 1µm antigen coupled particles than with the larger antigen coupled particles.

The cytokine release in these experiments serves as a proxy for T-cell expansion. In general, IFNy is produced by CD4+ T-cells (Th1 subclass) and CD8+ T-cells. IL17 and IL22 are mainly produced by pro-inflammatory Th17 CD4+ T-cells. Such cells are pro-inflammatory have been shown to assist in tumor eradication. The data suggest that the 1µm beads activate and cause expansion of Th1 CD4+ T-cells and CD8+ T-cells to the same degree as the other beads, with the added benefit of also activating and causing expansion of additional pro-inflammatory Th17 CD4+ T-cells and the less distinct but still pro-inflammatory double cytokine producing T-cells.

### Example 4: pilot study

### A pilot study utilising the method of the invention has been performed:

### (i) Identification of neo-antigen targets in urinary bladder cancer

Urinary bladder cancers display a high rate of mutations, thus expressing a large number of neo-antigens that could potentially be recognized as non-self by the immune system. As such, the inventors investigated tumor polymorphisms suitable as neo antigens and T-cells targets by mining mutation databases containing large repositories of potential neo-antigens that may be used to expand T-cells for immunotherapy.

The COSMIC database [27899578] contains mutation data for 4754 transitional cell carcinomas, both whole exome sequencing and hotspot-analyses. The inventors focused on urinary bladder cancer (UBC) and selected the 15 of the most prevalent mutations resulting in a single amino acid change, thus qualifying as a neo-antigen. The inventors also focused on polymorphisms in genes known to be associated with tumor pathogenesis such as kinases, growth factor receptors and cell cycle proteins. The chosen 15 mutations alone cover 73% of bladder cancer mutations found in COSMIC. Neo-peptides were designed as the mutated amino acid plus ten flanking amino acids to the C-terminal end and N-terminal end of the mutated one, resulting in a 21 aa peptide. To design neo-antigen constructs, three neo-antigen sequences were lined with two VVR-spacers, since the VVR motif is cleaved by cathepsin S in lysosomes, after translation, as a step in human leukocyte antigen presentation.

As an alternative, whole genome sequencing of a tumor from a patient with UBC is carried out in order to identify additional polymorphisms and new targets for immunotherapy. In RNA sequencing of tumors can be carried out to verify the presence of transcripts carrying the polymorphisms. Multiple reaction monitoring (MRM) mass spectrometry transitions can be used to quickly scan patients for expression of the most common neo-antigens at the protein level, tailoring the neo-antigens used for individual immunotherapy.

### (ii) Recombinant neo-antigen production

A highly efficient protocol for recombinant protein production has been developed. Synthetic genes are designed, purchased and sub-cloned into ready-to-use vectors adjusted for different cloning and expression conditions. Neo-antigens are expressed in E. coli, purified by Ni-IMAC chromatography and prepared for efficient antigen presentation by coupling to Dynabeads after testing for purity and endotoxin levels. With the established methodological platform, cloning and expression of recombinant proteins can be executed within a few weeks.

### (iii) T-cell activation and expansion by neo-antigens

Nine neo-antigens were identified bioinformatically, as described above. The designed neo-antigens were based on genes that harbor reported urinary bladder cancer associated mutations such as FGFR3 and p53. In a pilot experiment using a construct comprising 3 neo-antigens, the inventors were able to identify IFN-y producing T-cells from blood of a patient with urinary bladder cancer by FluoroSpot, demonstrating the validity of the neo-antigen approach.

For the same patient having urinary bladder cancer, activation of T-cells was performed using the predicted neo-antigen peptides NA1-9. Proliferation was seen in response to NA 1, 3, 5, 7 and 8. Figure 2A displays the number of cell in the culture over time (PB = Peripheral blood). The arrow in Figure 2A indicates the time of re-stimulation (i.e. the time when the T-cell sample was contacted with a second batch of antigen-presenting cells contacted with phaogcytosable particles under conditions allowing specific activation of anti-tumor T-cells in response to antigen presented by the antigen-presenting cell). Figure 2B shows the %CD4+/total T-cells. Figure 2C shows the T-bet expression in CD4, and in Figure 2D, shows the expression of granzyme B and perforin in CD8+ T-cells.

The expanded T-cells express the transcription factor Tbet and high levels of the effector molecules perforin and granzyme B (GZB).

The method of the invention has also been used on cells from a patient with disseminated colon cancer from whom sequenced tumor data were available. The patient displayed two polymorphisms in p53, one known and one novel. The patient also displayed a mutation in PIK3CA. A tumor neo-antigen construct was designed, expressed and purified, based on the specific mutations in the tumor data for the patient. The neo-antigen construct was used for expanding cells, resulting in a neo-antigen specific response. Peripheral blood mononuclear cells (PBMCs) were used for culture. Figure 3 shows the percent among T-cells (small squares) and total number (large squares) of CD4+ T-cells, as well as proliferating CD4+ cells (circles) in the T-sample before and during the expansion. Figure 4A shows the number of cell in the culture over time (the red line shows the personalized peptide, and pink and orange lines displays control cultures predicted peptides with the same protein but with the predicted mutations (NA1, 3, 4 and 5)). Figure 4B shows the %CD4+/total T-cells. The arrows in Figures 4A and 4B indicates the time of re-stimulation (i.e. the time when the T-cell sample was contacted with a second batch of antigen-presenting cells contacted with phaogcytosable particles under conditions allowing specific activation of anti-tumor T-cells in response to antigen presented by the antigen-presenting cell). Figure 4C visualizes an analysis performed with the Barnes-Hut Stochastic Neighbor Embedding (BH-SNE) algorithm for CD4 T-cells, where all cells in the samples are clustered on a 2-dimentional map according to the similarity in expression intensity according to a set of chosen markers, here CD28, CD57, T-bet, GATA-3, Perforin, Granzyme B (GZB), Ki-67 and PD-1.

The expression of the proliferation marker Ki67 and the number of T-cells increased in the expansion, and the expression of important markers for anti-tumor activity, such as Tbet, perforin and granzyme B, increased on both CD4+ and CD8+ cells over the 14-day culture period. The percentage of CD8+ T-cells decreased to around 10% of CD4+ T-cells, but the total number of CD8+ cells increased.

The whole process from receiving sequence data to analysis of expanded cells can be completed in 4-5 weeks.

These results show that there was a tailored Th1 neo-antigen response. Thus, the inventors have demonstrated that predicted and sequence verified neo-antigens can be designed and used for T-cell activation and expansion.

## Claims

1. A method for the expansion of anti-tumor T-cells from a subject with cancer, comprising the steps of:
a) providing a phagocytosable particle, the particle having paramagnetic properties and having one or more tumor neoantigenic constructs tightly associated thereto, wherein the tumor neoantigenic construct comprises an amino-acid sequence comprising at least one mutated amino acid known or suspected to be associated with the cancer in the subject, or a mutated amino-acid sequence known or suspected to be expressed in a cancer cell in the subject;
b) providing a viable antigen-presenting cell;
c) contacting the particle with the antigen-presenting cell *in vitro* under conditions allowing phagocytosis of the particle by the antigen-presenting cell;
d) providing a T-cell sample comprising viable T-cells from the subject;
e) contacting the T-cell sample with the antigen-presenting cell contacted with the particle *in vitro* under conditions allowing specific activation and expansion of anti-tumor T-cells in response to antigen presented by the antigen-presenting cell.

2. The method according to claim 1, wherein the particle with the associated polypeptide construct of step (a) has been subjected to a sterilising wash resulting in an aseptic particle with the associated polypeptide construct, and preferably a sterile particle with the associated polypeptide.

3. The method according to claim 1 or claim 2, wherein the method further comprises:
(f) removing the particle from the T-cell sample; and/or
(g) removing the particle and antigen-presenting cell from the T-cell sample.

4. The method according to claim 3, wherein the T-cell sample in steps d) to g) is free from anti-CD3 antibody and/or anti-CD28 antibody.

5. The method according to any of the preceding claims, wherein the antigen-presenting cell is from the subject.

6. The method according to any of the preceding claims, wherein the tumor neoantigenic construct comprises one or more covalently linked peptides, wherein one or more of the covalently linked peptides comprises an amino-acid sequence comprising at least one mutated amino acid known or suspected to be associated with a cancer in a subject, preferably the tumor neoantigenic construct comprises one or more covalently linked peptides, wherein one or more of the covalently linked peptides comprises an amino-acid sequence corresponding to a mutated amino-acid sequence known or suspected to be expressed in a cancer cell in a subject.

7. The method according to claim 6, wherein the tumor neoantigenic construct comprises three or more covalently linked peptides (for example three, four or five covalently linked peptides), and/or wherein each peptide of the tumor neoantigenic construct comprises 10 to 25 amino-acids.

8. The method according to any of the preceding claims, wherein two or more tumor neoantigenic constructs are tightly associated with the particle, and each tumor neoantigenic construct may have the same polypeptide sequence or may have different polypeptide sequences.

9. The method according to any of the preceding claims, wherein the anti-tumor T-cells expanded in the method are CD4+ helper and/or CD8+ T-cells, and/or wherein the T-cell sample comprises CD4+ helper and/or CD8+ T-cells and/or wherein the T-cell sample is derived from a tumor, preferably a lymphatic vessel in a tumor.

10. The method according to any of the preceding claims, wherein the cancer is a solid cancer, for example a cancer selected from breast cancer, colon cancer, liver cancer, lung cancer (non-small cell and small cell), lung carcinoid tumor, pancreatic cancer, prostate cancer, ovarian cancer and urinary bladder cancer.

11. The method according to any of the preceding claims, wherein the particle has a largest dimension of less than 5.6 µm, preferably less than 4 µm, more preferably less than 3 µm, even more preferably from 0.5 to 2 µm , or most preferably about 1 µm .

12. The method according to any of claims 2 to 11, wherein the sterilising wash or denaturing wash involves subjecting the particle with the associated polypeptide to a high pH, such as at least pH 13, more preferably at least pH 14, most preferably at least pH 14.3; or wherein the sterilising wash involves subjecting the particle with the associated polypeptide to a low pH, for example less than pH 2, preferably less than pH 1, and/or
wherein the sterilising wash or denaturing wash involves subjecting the particle with the associated polypeptide to a high temperature, such as at least 90°C, more preferably at least 92°C, most preferably at least 95°C, and/or
wherein the sterilising wash or denaturing wash involves subjecting the particle with the associated polypeptide to a sterilising/denaturing agent, such as urea or guanidine hydrochloride at a sufficient concentration, such as at least 5M, 6M, 7M or 8M.

13. The method according to any of claims any of claims 1 to 12, wherein the particle is a polymer particle, preferably a polystyrene particle.

14. The method according to any of the preceding claims, wherein one or more tumor neoantigenic constructs is covalently linked to the particle.

15. The method according to any of the preceding claims, wherein the antigen-presenting cell and the T-cell sample are derived from the same subject, and more preferably derived from the same blood sample, for example the antigen- presenting cell and the T-cell sample are derived from a PBMC-sample from the same subject.

16. A composition comprising anti-tumor T-cells from a subject with cancer, wherein the composition comprises a mixture of CD4+ helper cells and CD8+ T-cells and is produced by a method comprising the steps of:
a) providing a phagocytosable particle, the particle having paramagnetic properties and having one or more tumor neoantigenic constructs tightly associated thereto, wherein the tumor neoantigenic construct comprises an amino-acid sequence comprising at least one mutated amino acid known or suspected to be associated with the cancer in the subject, or a mutated amino-acid sequence known or suspected to be expressed in a cancer cell in the subject;
b) providing a viable antigen-presenting cell;
c) contacting the particle with the antigen-presenting cell *in vitro* under conditions allowing phagocytosis of the particle by the antigen-presenting cell;
d) providing a T-cell sample comprising viable T-cells from the subject;
e) contacting the T-cell sample with the antigen-presenting cell contacted with the particle *in vitro* under conditions allowing specific activation and expansion of anti-tumor T-cells in response to antigen presented by the antigen-presenting cell.
(f) removing the particle from the T-cell sample and/or removing the particle and antigen-presenting cell from the T-cell sample;
wherein the T-cell sample in steps (d) to (f) is free from anti-CD3 and/or anti-CD28 antibodies.

17. The composition of claim 16 for use in the treatment of cancer in a subject.

18. The method of any one of claims 1 to 15, further comprising immediately after step (e):
a1) providing a second phagocytosable particle, having one or more tumor neoantigenic constructs tightly associated thereto, wherein the tumor neoantigenic construct comprises an amino-acid sequence comprising at least one mutated amino acid known or suspected to be associated with the cancer in the subject, or a mutated amino-acid sequence known or
suspected to be expressed in a cancer cell in the subject;
b1) providing a second viable antigen-presenting cell;
c1) contacting the second particle with the second antigen-presenting cell *in vitro* under conditions allowing phagocytosis of the particle by the antigen-presenting cell; and
e1) contacting the T-cell sample with the second antigen-presenting cell contacted with the second particle *in vitro* under conditions allowing specific activation and expansion of anti-tumor T-cells in response to antigen presented by the antigen-presenting cell; and
wherein preferably the second phagocytosable particle, having one or more tumor neoantigenic constructs tightly associated thereto, of step (a1) is the same as the phagocytosable particle, having one or more tumor neoantigenic constructs tightly associated thereto, of step (a); and/or the second viable antigen-presenting cell of step (b1) is the same as the viable antigen-presenting cell of step (b).

## Patentansprüche

1. Verfahren zur Vermehrung von Antitumor-T-Zellen eines Subjekts mit Krebs, enthaltend die folgenden Schritte:
a) Bereitstellen eines phagozytierbaren Partikels, wobei der Partikel paramagnetische Eigenschaften hat und ein oder mehrere neoantigene Konstrukte gegen Tumor an den Partikel gebunden hat, wobei das neoantigene Konstrukt gegen Tumor eine Aminosäuresequenz enthält, die mindestens eine mutierte Aminosäure, von der bekannt ist oder von der vermutet wird, dass sie in Verbindung mit dem Krebs in dem Subjekt steht, enthält, oder eine mutierte Aminosäuresequenz, von der bekannt ist oder vermutet wird, dass sie in einer Krebszelle in dem Subjekt exprimiert wird, enthält;
b) Breitstellen einer lebensfähigen Zelle, die das Antigen präsentiert;
c) in Kontakt bringen des Partikels mit der Zelle, die das Antigen präsentiert, *in vitro* unter Bedingungen, die die Phagozytose des Partikels durch die Zelle, die das Antigen präsentiert, ermöglichen;
d) Bereitstellen einer T-Zellen-Probe, die lebensfähige T-Zellen des Subjekts enthält;
e) in Kontakt bringen der T-Zellen-Probe mit der Zelle, die das Antigen präsentiert, die mit dem Partikel *in vitro* unter Bedingungen, die die spezifische Aktivierung der Zelle ermöglichen, in Kontakt gebracht wurde, und Vermehrung der Antitumor-T-Zellen als Reaktion auf das Antigen, das von der Zelle, die das Antigen präsentiert, präsentiert wird.

2. Verfahren nach Anspruch 1, wobei der Partikel mit dem assoziierten Polypeptidkonstrukt aus Schritt (a) einer sterilisierenden Spülung ausgesetzt wurde, was in einem aseptischen Partikel mit dem assoziierten Polypeptidkonstrukt, und vorzugsweise in einem sterilen Partikel mit dem assoziierten Polypeptid resultiert.

3. Verfahren nach Anspruch 1 oder 2, wobei das Verfahren weiter enthält:
f) Entfernen des Partikels aus der T-Zellen-Probe; und/oder
g) Entfernen des Partikels und der Zelle, die das Antigen präsentiert, aus der T-Zellen-Probe.

4. Verfahren nach Anspruch 3, wobei die T-Zellen-Probe in Schritten d) bis g) frei von anti-CD3 Antikörper und/oder anti-CD28 Antikörper ist.

5. Verfahren nach einem der vorhergehenden Ansprüche, wobei die Zelle, die das Antigen präsentiert, von dem Subjekt ist.

6. Verfahren nach einem der vorhergehenden Ansprüche, wobei das neoantigene Konstrukt gegen Tumor ein oder mehrere kovalent verbundene Peptide enthält, wobei ein oder mehrere der kovalent verbundenen Peptide eine Aminosäuresequenz enthalten, die mindestens eine mutierte Aminosäure, von der bekannt ist oder von der vermutet wird, dass sie in Verbindung mit dem Krebs in dem Subjekt steht, enthält, vorzugsweise enthält das neoantigene Konstrukt gegen Tumor ein oder mehrere kovalent verbundene Peptide, wobei ein oder mehrere der kovalent verbundenen Peptide eine Aminosäuresequenz enthalten, die mit einer mutierten Aminosäuresequenz, von der bekannt ist oder von der vermutet wird, dass sie in Verbindung mit dem Krebs in dem Subjekt steht, übereinstimmt.

7. Verfahren nach Anspruch 6, wobei das neoantigene Konstrukt gegen Tumor drei oder mehrere kovalent verbundene Peptide (zum Beispiel drei, vier oder fünf kovalent verbundene Peptide) enthält, und/oder wobei jedes Peptid des neoantigenen Konstrukts gegen Tumor 10 bis 25 Aminosäuren enthält.

8. Verfahren nach einem der vorhergehenden Ansprüche, wobei zwei oder mehrere neoantigene Konstrukte gegen Tumor eng mit dem Partikel assoziiert sind, und jedes neoantigene Konstrukt gegen Tumor dieselbe oder verschiedene Polypeptidsequenzen haben kann.

9. Verfahren nach einem der vorhergehenden Ansprüche, wobei die Antitumor-T-Zellen, die im Verfahren vermehrt werden, CD4+ Helferzellen und/oder CD8+ T-Zellen sind, und/oder wobei die T-Zellen-Probe CD4+ Helferzellen und/oder CD8+ T-Zellen enthält und/oder wobei die T-Zellen-Probe von einem Tumor, vorzugsweise lymphatischem Gefäß in einem Tumor, abgeleitet ist.

10. Verfahren nach einem der vorhergehenden Ansprüche, wobei der Krebs ein fester Krebs ist, zum Beispiel ein Krebs ausgewählt aus Brustkrebs, Darmkrebs, Leberkrebs, Lungenkrebs (nicht-kleinzellig und kleinzellig), karzinoider Lungentumor, Bauchspeicheldrüsenkrebs, Prostatakrebs, Eierstockkrebs und Harnblasenkrebs.

11. Verfahren nach einem der vorhergehenden Ansprüche, wobei der Partikel eine größte Dimension von weniger als 5.6 µm, vorzugsweise von weniger als 4 µm, weiter vorzugsweise von weniger als 3 µm, weiter vorzugsweise von 0.5 bis 2 µm, oder am meisten bevorzugt von etwa 1 µm hat.

12. Verfahren nach einem der Ansprüche 2 bis 11, wobei die sterilisierende Spülung oder denaturierende Spülung beinhaltet den Partikel mit dem assoziierten Polypeptid einem hohen pH, wie etwa mindestens pH 13, vorzugsweise mindestens pH 14, am meisten bevorzugt mindestens pH 14.3 auszusetzen; oder wobei die sterilisierende Spülung beinhaltet den Partikel mit dem assoziierten Polypeptid einem niedrigen pH, zum Beispiel weniger als pH 2, vorzugsweise weniger als pH 1 auszusetzen, und/oder
wobei die sterilisierende Spülung oder denaturierende Spülung beinhaltet den Partikel mit dem assoziierten Polypeptid einer hohen Temperatur, wie etwa mindestens 90°C, vorzugsweise mindestens 92 °C, am meisten bevorzugt mindestens 95°C auszusetzen, und/oder
wobei die sterilisierende Spülung oder denaturierende Spülung beinhaltet den Partikel mit dem assoziierten Polypeptid einem sterilisierenden/denaturierenden Wirkstoff, wie etwa Harnsäure oder Guanidinhydrochlorid in einer ausreichenden Konzentration, wie etwa mindestens 5 M, 6 M, 7 M oder 8 M auszusetzen.

13. Verfahren nach einem der Ansprüche 1 bis 12, wobei der Partikel ein Polymerpartikel, vorzugsweise ein Polystyrolpartikel, ist.

14. Verfahren nach einem der vorhergehenden Ansprüche, wobei ein oder mehrere der neoantigenen Konstrukte gegen Tumor kovalent an den Partikel gebunden sind.

15. Verfahren nach einem der vorhergehenden Ansprüche, wobei die Zelle, die das Antigen präsentiert, und die T-Zellen-Probe von demselben Subjekt abgeleitet sind, und vorzugsweise aus derselben Blutprobe abgeleitet sind, zum Beispiel sind die Zelle, die das Antigen präsentiert, und die T-Zellen-Probe aus einer PBMC-Probe desselben Subjekts abgeleitet.

16. Komposition, die Antitumor-T-Zellen von einem Subjekt mit Krebs enthält, wobei die Komposition eine Mischung aus CD4+ Helferzellen und CD8+ T-Zellen enthält und durch ein Verfahren, das die folgenden Schritte enthält, hergestellt wird:
a) Bereitstellen eines phagozytierbaren Partikels, wobei der Partikel paramagnetische Eigenschaften hat und ein oder mehrere neoantigene Konstrukte gegen Tumor an den Partikel gebunden hat, wobei das neoantigene Konstrukt gegen Tumor eine Aminosäuresequenz enthält, die mindestens eine mutierte Aminosäure, von der bekannt ist oder von der vermutet wird, dass sie in Verbindung mit dem Krebs in dem Subjekt steht, enthält, oder eine mutierte Aminosäuresequenz, von der bekannt ist oder vermutet wird, dass sie in einer Krebszelle in dem Subjekt exprimiert wird, enthält;
b) Breitstellen einer lebensfähigen Zelle, die das Antigen präsentiert;
c) in Kontakt bringen des Partikels mit der Zelle, die das Antigen präsentiert, *in vitro* unter Bedingungen, die die Phagozytose des Partikels durch die Zelle, die das Antigen präsentiert, ermöglichen;
d) Bereitstellen einer T-Zellen-Probe, die lebensfähige T-Zellen des Subjekts enthält;
e) in Kontakt bringen der T-Zellen-Probe mit der Zelle, die das Antigen präsentiert, die mit dem Partikel *in vitro* unter Bedingungen, die die spezifische Aktivierung der Zelle ermöglichen, in Kontakt gebracht wurde, und Vermehrung der Antitumor-T-Zellen als Reaktion auf das Antigen, das von der Zelle, die das Antigen präsentiert, präsentiert wird;
f) Entfernen des Partikels aus der T-Zellen-Probe und/oder Entfernen des Partikels und der Zelle, die das Antigen präsentiert, aus der T-Zellen-Probe;
wobei die T-Zellen-Probe in Schritten (d) bis (f) frei von anti-CD3 Antikörper und/oder anti-CD28 Antikörper ist.

17. Komposition nach Anspruch 16 zur Verwendung in der Behandlung von Krebs in einem Subjekt.

18. Verfahren nach einem der Ansprüche 1 bis 15 weiter enthaltend direkt nach Schritt (e):
a1) Bereitstellen eines zweiten phagozytierbaren Partikels, der ein oder mehrere neoantigene Konstrukte gegen Tumor an den Partikel gebunden hat, wobei das neoantigene Konstrukt gegen Tumor eine Aminosäuresequenz enthält, die mindestens eine mutierte Aminosäure, von der bekannt ist oder von der vermutet wird, dass sie in Verbindung mit dem Krebs in dem Subjekt steht, enthält, oder eine mutierte Aminosäuresequenz, von der bekannt ist oder vermutet wird, dass die in einer Krebszelle in dem Subjekt exprimiert wird, enthält;
b1) Bereitstellen einer zweiten lebensfähigen Zelle, die das Antigen präsentiert;
c1) in Kontakt bringen des zweiten Partikels mit der zweiten Zelle, die das Antigen präsentiert, *in vitro* unter Bedingungen, die die Phagozytose des Partikels durch die Zelle, die das Antigen präsentiert, ermöglichen; und
e1) in Kontakt bringen der T-Zellen-Probe mit der zweiten Zelle, die das Antigen präsentiert, die mit dem zweiten Partikel *in vitro* unter Bedingungen, die die spezifische Aktivierung der Zelle ermöglichen, in Kontakt gebracht wurde, und Vermehrung der Antitumor-T-Zellen als Reaktion auf das Antigen, das von der Zelle, die das Antigen präsentiert, präsentiert wird; und wobei vorzugsweise der zweite phagozytierbare Partikel, der ein oder mehrere neoantigene Konstrukte gegen Tumor an den Partikel gebunden hat, aus Schritt (a1) gleich wie der phagozytierbare Partikel, der ein oder mehrere neoantigene Konstrukte gegen Tumor an den Partikel gebunden hat, aus Schritt (a) ist; und/oder die zweite lebensfähige Zelle, die das Antigen präsentiert, aus Schritt (b 1) gleich wie die lebensfähige Zelle, die das Antigen präsentiert, aus Schritt (b) ist.

## Revendications

1. Procédé d'expansion de lymphocytes T antitumoraux d'un sujet atteint d'un cancer, comprenant les étapes suivantes :
a) fournir une particule phagocytable, la particule ayant des propriétés paramagnétiques et ayant une ou plusieurs constructions néoantigéniques tumorales étroitement associées à celle-ci, dans lequel la construction néoantigénique tumorale comprend une séquence d'acides aminés comprenant au moins un acide aminé muté connu ou suspecté d'être associé au cancer chez le sujet, ou une séquence d'acides aminés mutés connue ou suspectée d'être exprimée dans une cellule cancéreuse chez le sujet ;
b) fournir une cellule de présentation d'antigène viable ;
c) mettre en contact la particule avec la cellule de présentation d'antigène *in vitro* dans des conditions permettant la phagocytose de la particule par la cellule de présentation d'antigène ;
d) fournir un échantillon de lymphocytes T comprenant des lymphocytes T viables provenant du sujet ;
e) mettre en contact l'échantillon de lymphocytes T avec la cellule de présentation d'antigène mise en contact avec la particule *in vitro* dans des conditions permettant l'activation spécifique et l'expansion des lymphocytes T antitumoraux en réponse à l'antigène présenté par la cellule de présentation d'antigène.

2. Procédé selon la revendication 1, dans lequel la particule avec la construction polypeptidique associée de l'étape (a) a été soumise à un lavage stérilisant résultant en une particule aseptique avec la construction polypeptidique associée, et de préférence une particule stérile avec le polypeptide associé.

3. Procédé selon la revendication 1 ou la revendication 2, dans lequel le procédé comprend en outre :
(f) le retrait de la particule de l'échantillon de lymphocytes T ; et/ou
(g) le retrait de la particule et de la cellule de présentation d'antigène de l'échantillon de lymphocytes T.

4. Procédé selon la revendication 3, dans lequel l'échantillon de lymphocytes T des étapes d) à g) est exempt d'anticorps anti-CD3 et/ou d'anticorps anti-CD28.

5. Procédé selon l'une quelconque des revendications précédentes, dans lequel la cellule de présentation d'antigène provient du sujet.

6. Procédé selon l'une quelconque des revendications précédentes, dans lequel la construction néoantigénique tumorale comprend un ou plusieurs peptides liés de manière covalente, dans lequel un ou plusieurs des peptides liés de manière covalente comprend une séquence d'acides aminés comprenant au moins un acide aminé muté connu ou suspecté d'être associé à un cancer chez un sujet, de préférence, la construction néoantigénique tumorale comprend un ou plusieurs peptides liés de manière covalente, dans lequel un ou plusieurs des peptides liés de manière covalente comprend une séquence d'acides aminés correspondant à une séquence d'acides aminés mutée connue ou suspectée d'être exprimée dans une cellule cancéreuse d'un sujet.

7. Procédé selon la revendication 6, dans lequel la construction néoantigénique tumorale comprend trois peptides liés de manière covalente ou plus (par exemple trois, quatre ou cinq peptides liés de manière covalente), et/ou dans lequel chaque peptide de la construction néoantigénique tumorale comprend 10 à 25 acides aminés.

8. Procédé selon l'une quelconque des revendications précédentes, dans lequel deux constructions néoantigéniques tumorales ou plus sont étroitement associées à la particule, et chaque construction néoantigénique tumorale peut avoir la même séquence polypeptidique ou peut avoir des séquences polypeptidiques différentes.

9. Procédé selon l'une quelconque des revendications précédentes, dans lequel les lymphocytes T antitumoraux expansés dans le procédé sont des lymphocytes T CD4+ auxiliaires et/ou CD8+, et/ou dans lequel l'échantillon de lymphocytes T comprend des lymphocytes T CD4+ auxiliaires et/ou CD8+ et/ou dans lequel l'échantillon de lymphocytes T est dérivé d'une tumeur, de préférence d'un vaisseau lymphatique d'une tumeur.

10. Procédé selon l'une quelconque des revendications précédentes, dans lequel le cancer est un cancer solide, par exemple un cancer choisi parmi le cancer du sein, le cancer du côlon, le cancer du foie, le cancer du poumon (non à petites cellules et à petites cellules), la tumeur carcinoïde du poumon, le cancer du pancréas, le cancer de la prostate, le cancer de l'ovaire et le cancer de la vessie.

11. Procédé selon l'une quelconque des revendications précédentes, dans lequel la particule a une plus grande dimension inférieure à 5,6 pm, de préférence inférieure à 4 pm, plus préférentiellement inférieure à 3 pm, encore plus préférentiellement de 0,5 à 2 pm, ou le plus préférentiellement d'environ 1 pm.

12. Procédé selon l'une quelconque des revendications 2 à 11, dans lequel le lavage stérilisant ou le lavage dénaturant consiste à soumettre la particule avec le polypeptide associé à un pH élevé, tel qu'au moins pH 13, de préférence au moins pH 14, le plus préférentiellement au moins pH 14,3 ; ou dans lequel le lavage stérilisant consiste à soumettre la particule avec le polypeptide associé à un pH faible, par exemple inférieur à pH 2, de préférence inférieur à pH 1 et/ou
dans lequel le lavage stérilisant ou le lavage dénaturant consiste à soumettre la particule avec le polypeptide associé à une température élevée, telle qu'au moins 90° C, de préférence au moins 92° C, le plus préférentiellement au moins 95° C, et/ou
dans lequel le lavage stérilisant ou le lavage dénaturant consiste à soumettre la particule avec le polypeptide associé à un agent stérilisant/dénaturant, tel que l'urée ou le chlorhydrate de guanidine à une concentration suffisante, telle qu'au moins 5M, 6M, 7M ou 8M.

13. Procédé selon l'une quelconque des revendications 1 à 12, dans lequel la particule est une particule polymère, de préférence une particule de polystyrène.

14. Procédé selon l'une quelconque des revendications précédentes, dans lequel une ou plusieurs constructions néoantigéniques tumorales sont liées de manière covalente à la particule.

15. Procédé selon l'une quelconque des revendications précédentes, dans lequel la cellule de présentation d'antigène et l'échantillon de lymphocytes T proviennent du même sujet, et plus préférablement du même échantillon de sang, par exemple la cellule de présentation d'antigène et l'échantillon de lymphocytes T proviennent d'un échantillon de CMSP du même sujet.

16. Composition comprenant des lymphocytes T antitumoraux provenant d'un sujet atteint d'un cancer, dans laquelle la composition comprend un mélange de cellules CD4+ auxiliaires et de lymphocytes T CD8+ et est produite par un procédé comprenant les étapes suivantes :
a) fournir une particule phagocytable, la particule ayant des propriétés paramagnétiques et ayant une ou plusieurs constructions néoantigéniques tumorales étroitement associées à celle-ci, dans laquelle la construction néoantigénique tumorale comprend une séquence d'acides aminés comprenant au moins un acide aminé muté connu ou suspecté d'être associé au cancer chez le sujet, ou une séquence d'acides aminés mutés connue ou suspectée d'être exprimée dans une cellule cancéreuse chez le sujet ;
b) fournir une cellule de présentation d'antigène viable ;
c) mettre en contact la particule avec la cellule de présentation d'antigène *in vitro* dans des conditions permettant la phagocytose de la particule par la cellule de présentation d'antigène ;
d) fournir un échantillon de lymphocytes T comprenant des lymphocytes T viables provenant du sujet ;
e) mettre en contact l'échantillon de lymphocytes T avec la cellule de présentation d'antigène mise en contact avec la particule *in vitro* dans des conditions permettant l'activation spécifique et l'expansion des lymphocytes T antitumoraux en réponse à l'antigène présenté par la cellule de présentation d'antigène.
(f) retirer la particule de l'échantillon de lymphocytes T et/ou retirer la particule et la cellule de présentation d'antigène de l'échantillon de lymphocytes T ;
dans laquelle l'échantillon de lymphocytes T des étapes (d) à (f) est exempt d'anticorps anti-CD3 et/ou anti-CD28.

17. Composition selon la revendication 16 destinée à être utilisée dans le traitement du cancer chez un sujet.

18. Procédé selon l'une quelconque des revendications 1 à 15, comprenant en outre immédiatement après l'étape (e) :
a1) la fourniture d'une seconde particule phagocytable, à laquelle sont étroitement associées une ou plusieurs constructions néoantigéniques tumorales, dans lequel la construction néoantigénique tumorale comprend une séquence d'acides aminés comprenant au moins un acide aminé muté connu ou suspecté d'être associé au cancer chez le sujet, ou une séquence d'acides aminés mutés connue ou suspectée d'être exprimée dans une cellule cancéreuse chez le sujet ;
b1) la fourniture d'une seconde cellule de présentation d'antigène viable ;
c1) la mise en contact de la seconde particule avec la seconde cellule de présentation d'antigènes *in vitro* dans des conditions permettant la phagocytose de la particule par la cellule de présentation d'antigène ; et
e1) la mise en contact de l'échantillon de lymphocytes T avec la seconde cellule de présentation d'antigène en contact avec la seconde particule *in vitro* dans des conditions permettant l'activation spécifique et l'expansion des lymphocytes T antitumoraux en réponse à l'antigène présenté par la cellule de présentation d'antigène ; et
dans lequel, de préférence, la seconde particule phagocytable, ayant une ou plusieurs constructions néoantigéniques tumorales
étroitement associées à celle-ci, de l'étape (a1) est la même que la particule phagocytable, ayant une ou plusieurs constructions néoantigéniques tumorales étroitement associées à celle-ci, de l'étape (a) ; et/ou la seconde cellule de présentation d'antigène viable de l'étape (b1) est la même que la cellule de présentation d'antigène viable de l'étape (b).
